# EUROPEAN PATENT SPECIFICATION

(11) **EP 1 407 030 B1**
(45) Date of publication and mention of the grant of the patent: **06.04.2011**
(21) Application number: 01996596.1
(22) Date of filing: 07.11.2001
(51) Int. Cl.: C12N 15/62

(54) **METHODS FOR LARGE SCALE PRODUCTION OF RECOMBINANT DNA-DERIVED TPA OR K2S MOLECULES**
VERFAHREN ZUR PRODUKTION VON REKOMBINANTEN, DNA-ABGELEITETEN TPA- ODER K2S-MOLEKÜLEN IN GROSSEM MASSSTAB
PROCEDES DE PRODUCTION A GRANDE ECHELLE DE MOLECULES K2S TPAOR DERIVEES D'ADN RECOMBINANT

(30) Priority: 14.11.2000 GB 0027779
(43) Date of publication of application: 14.04.2004
(73) Proprietor: Boehringer Ingelheim International GmbH, 55216 Ingelheim am Rhein (DE)
(72) Inventor: MANOSROI, Jiradej, Nong Hoi, Muang Chiang Mai 50000 (TH); MANOSROI, Aranya, Nong Hoi, Muang Chiang Mai 50000 (TH); TAYAPIWATANA, Chatchai, Bangkok 10120 (TH); GOETZ, Friedrich, 72076 Tübingen (DE); WERNER, Rolf-Günther, 88400 Biberach an der Riss (DE)
(86) International application number: PCT/EP2001/012857
(87) International publication number: WO 2002/040650

(56) References cited:
- EP-A- 0 302 456
- EP-A- 1 048 732
- EP-A- 1 077 263
- WO-A-97/38123
- WO-A-98/54199
- US-A- 6 083 715
- HUA ZI-CHUN ET AL: "Synthesis and expression of a gene from Kringle-2 domain of tissue plasminogen activator in E. coli." SCIENCE IN CHINA SERIES B CHEMISTRY LIFE SCIENCES & EARTH SCIENCES, vol. 37, no. 6, 1994, pages 667-676, XP002196158 ISSN: 1001-652X
- MANOSROI JIRADEJ ET AL: "Secretion of active recombinant human tissue plasminogen activator derivatives in Escherichia coli." APPLIED AND ENVIRONMENTAL MICROBIOLOGY, vol. 67, no. 6, June 2001 (2001-06), pages 2657-2664, XP002195206 ISSN: 0099-2240
- SCOTT CLEARY ET AL: "Purification and characterization of tissue plasminogen activator kringle-2 domain expressed in Escherichia coli" BIOCHEMISTRY, AMERICAN CHEMICAL SOCIETY. EASTON, PA, US, vol. 28, no. 4, 21 February 1989 (1989-02-21), pages 1884-1891, XP002101498 ISSN: 0006-2960
- QIU JI ET AL: "Expression of active human tissue-type plasminogen activator in Escherichia coli." APPLIED AND ENVIRONMENTAL MICROBIOLOGY, vol. 64, no. 12, December 1998 (1998-12), pages 4891-4896, XP002195207 ISSN: 0099-2240
- WALDENSTROM M ET AL: "SYNTHESIS AND SECRETION OF A FIBRINOLYTICALLY ACTIVE TISSUE-TYPE PLASMINOGEN ACTIVATOR VARIANT IN ESCHERICHIA-COLI" GENE (AMSTERDAM), vol. 99, no. 2, 1991, pages 243-248, XP001063188 ISSN: 0378-1119
- LASTERS IGNACE ET AL: "Enzymatic properties of phage-displayed fragments of human plasminogen." EUROPEAN JOURNAL OF BIOCHEMISTRY, vol. 244, no. 3, 1997, pages 946-952, XP001068905 ISSN: 0014-2956

### SEQUENCE LISTING

<110> Boehringer Ingelheim International GmbH
<120> Methods for large scale production of recombinant DNA-derived tPA or K2S molecules
<130> case 1-1170
<140>
   <141>
<150> GB 0027779.8
   <151> 2000-11-14
<160> 25
<170> PatentIn Ver. 2.1
<210> 1
   <211> 18
   <212> DNA
   <213> Artificial Sequence
<220>
   <223> Description of Artificial Sequence: coding sequence of N-terminal part of K2S protein
<400> 1
   tctgagggaa acagtgac 18
<210> 2
   <211> 1128
   <212> DNA
   <213> Artificial Sequence
<220>
   <223> Description of Artificial Sequence: coding sequence for OmpA-K2S fusion protein
<400> 2
   atgaaaaaga cagctatcgc gattgcagtg gcactggctg gtttcgctac cgtggcccag 60
   gcggcctctg agggaaacag tgactgctac tttgggaatg ggtcagccta ccgtggcacg 120
   cacagcctca ccgagtcggg tgcctcctgc ctcccgtgga attccatgat cctgataggc 180
   aaggtttaca cagcacagaa ccccagtgcc caggcactgg gcctgggcaa acataattac 240
   tgccggaatc ctgatgggga tgccaagccc tggtgccacg tgctgaagaa ccgcaggctg 300
   acgtgggagt actgtgatgt gccctcctgc tccacctgcg gcctgagaca gtacagccag 360
   cctcagtttc gcatcaaagg agggctcttc gccgacatcg cctcccaccc ctggcaggct 420
   gccatctttg ccaagcacag gaggtcgccc ggagagcggt tcctgtgcgg gggcatactc 480
   atcagctcct gctggattct ctctgccgcc cactgcttcc aggagaggtt tccgccccac 540
   cacctgacgg tgatcttggg cagaacatac cgggtggtcc ctggcgagga ggagcagaaa 600
   tttgaagtcg aaaaatacat tgtccataag gaattcgatg atgacactta cgacaatgac 660
   attgcgctgc tgcagctgaa atcggattcg tcccgctgtg cccaggagag cagcgtggtc 720
   cgcactgtgt gccttccccc ggcggacctg cagctgccgg actggacgga gtgtgagctc 780
   tccggctacg gcaagcatga ggccttgtct cctttctatt cggagcggct gaaggaggct 840
   catgtcagac tgtacccatc cagccgctgc acatcacaac atttacttaa cagaacagtc 900
   accgacaaca tgctgtgtgc tggagacact cggagcggcg ggccccaggc aaacttgcac 960
   gacgcctgcc agggcgattc gggaggcccc ctggtgtgtc tgaacgatgg ccgcatgact 1020
   ttggtgggca tcatcagctg gggcctgggc tgtggacaga aggatgtccc gggtgtgtac 1080
   acaaaggtta ccaactacct agactggatt cgtgacaaca tgcgaccg             1128
<210> 3
   <211> 66
   <212> DNA
   <213> Escherichia coli
<400> 3
   atgaaaaaga cagctatcgc gattgcagtg gcactggctg gtttcgctac cgtggcccag 60
   gcggcc                                                            66
<210> 4
   <211> 1065
   <212> DNA
   <213> Artificial Sequence
<220>
   <223> Description of Artificial Sequence: coding sequence for K2S protein
<400> 4
   tctgagggaa acagtgactg ctactttggg aatgggtcag cctaccgtgg cacgcacagc 60
   ctcaccgagt cgggtgcctc ctgcctcccg tggaattcca tgatcctgat aggcaaggtt 120
   tacacagcac agaaccccag tgcccaggca ctgggcctgg gcaaacataa ttactgccgg 180
   aatcctgatg gggatgccaa gccctggtgc cacgtgctga agaaccgcag gctgacgtgg 240
   gagtactgtg atgtgccctc ctgctccacc tgcggcctga gacagtacag ccagcctcag 300
   tttcgcatca aaggagggct cttcgccgac atcgcctccc acccctggca ggctgccatc 360
   tttgccaagc acaggaggtc gcccggagag cggttcctgt gcgggggcat actcatcagc 420
   tcctgctgga ttctctctgc cgcccactgc ttccaggaga ggtttccgcc ccaccacctg 480
   acggtgatct tgggcagaac ataccgggtg gtccctggcg aggaggagca gaaatttgaa 540
   gtcgaaaaat acattgtcca taaggaattc gatgatgaca cttacgacaa tgacattgcg 600
   ctgctgcagc tgaaatcgga ttcgtcccgc tgtgcccagg agagcagcgt ggtccgcact 660
   gtgtgccttc ccccggcgga cctgcagctg ccggactgga cggagtgtga gctctccggc 720
   tacggcaagc atgaggcctt gtctcctttc tattcggagc ggctgaagga ggctcatgtc 780
   agactgtacc catccagccg ctgcacatca caacatttac ttaacagaac agtcaccgac 840
   aacatgctgt gtgctggaga cactcggagc ggcgggcccc aggcaaactt gcacgacgcc 900
   tgccagggcg attcgggagg ccccctggtg tgtctgaacg atggccgcat gactttggtg 960
   ggcatcatca gctggggcct gggctgtgga cagaaggatg tcccgggtgt gtacacaaag 1020
   gttaccaact acctagactg gattcgtgac aacatgcgac cgtga                 1065
<210> 5
   <211> 1128
   <212> DNA
   <213> Artificial Sequence
<220>
   <223> Description of Artificial Sequence: coding sequence for OmpA-K2S fusion protein
<400> 5
   atgaaaaaga cagctatcgc gattgcagtg gcactggctg gtttcgctac cgtggcccag 60
   gcggcctctg agggaaacag tgactgctac tttgggaatg ggtcagccta ccgtggcacg 120
   cacagcctca ccgagtcggg tgcctcctgc ctcccgtgga attccatgat cctgataggc 180
   aaggtttaca cagcacagaa ccccagtgcc caggcactgg gcctgggcaa acataattac 240
   tgccggaatc ctgatgggga tgccaagccc tggtgccacg tgctgaagaa ccgcaggctg 300
   acgtgggagt actgtgatgt gccctcctgc tccacctgcg gcctgagaca gtacagccag 360
   cctcagtttc gcatcaaagg agggctcttc gccgacatcg cctcccaccc ctggcaggct 420
   gccatctttg ccaagcacag gaggtcgccc ggagagcggt tcctgtgcgg gggcatactc 480
   atcagctcct gctggattct ctctgccgcc cactgcttcc aggagaggtt tccgccccac 540
   cacctgacgg tgatcttggg cagaacatac cgggtggtcc ctggcgagga ggagcagaaa 600
   tttgaagtcg aaaaatacat tgtccataag gaattcgatg atgacactta cgacaatgac 660
   attgcgctgc tgcagctgaa atcggattcg tcccgctgtg cccaggagag cagcgtggtc 720
   cgcactgtgt gccttccccc ggcggacctg cagctgccgg actggacgga gtgtgagctc 780
   tccggctacg gcaagcatga ggccttgtct cctttctatt cggagcggct gaaggaggct 840
   catgtcagac tgtacccatc cagccgctgc acatcacaac atttacttaa cagaacagtc 900
   accgacaaca tgctgtgtgc tggagacact cggagcggcg ggccccaggc aaacttgcac 960
   gacgcctgcc agggcgattc gggaggcccc ctggtgtgtc tgaacgatgg ccgcatgact 1020
   ttggtgggca tcatcagctg gggcctgggc tgtggacaga aggatgtccc gggtgtgtac 1080
   acaaaggtta ccaactacct agactggatt cgtgacaaca tgcgaccg             1128
<210> 6
   <211> 66
   <212> DNA
   <213> Escherichia coli
<400> 6
   atgaaaaaga cagctatcgc gattgcagtg gcactggctg gtttcgctac cgtggcccag 60
   gcggcc                                                            66
<210> 7
   <211> 1065
   <212> DNA
   <213> Artificial Sequence
<220>
   <223> Description of Artificial Sequence: coding sequence for K2S protein
<400> 7
   tctgagggaa acagtgactg ctactttggg aatgggtcag cctaccgtgg cacgcacagc 60
   ctcaccgagt cgggtgcctc ctgcctcccg tggaattcca tgatcctgat aggcaaggtt 120
   tacacagcac agaaccccag tgcccaggca ctgggcctgg gcaaacataa ttactgccgg 180
   aatcctgatg gggatgccaa gccctggtgc cacgtgctga agaaccgcag gctgacgtgg 240
   gagtactgtg atgtgccctc ctgctccacc tgcggcctga gacagtacag ccagcctcag 300
   tttcgcatca aaggagggct cttcgccgac atcgcctccc acccctggca ggctgccatc 360
   tttgccaagc acaggaggtc gcccggagag cggttcctgt gcgggggcat actcatcagc 420
   tcctgctgga ttctctctgc cgcccactgc ttccaggaga ggtttccgcc ccaccacctg 480
   acggtgatct tgggcagaac ataccgggtg gtccctggcg aggaggagca gaaatttgaa 540
   gtcgaaaaat acattgtcca taaggaattc gatgatgaca cttacgacaa tgacattgcg 600
   ctgctgcagc tgaaatcgga ttcgtcccgc tgtgcccagg agagcagcgt ggtccgcact 660
   gtgtgccttc cccggcggga cctgcagctg ccggactgga cggagtgtga gctctccggc 720
   tacggcaagc atgaggcctt gtctcctttc tattcggagc ggctgaagga ggctcatgtc 780
   agactgtacc catccagccg ctgcacatca caacatttac ttaacagaac agtcaccgac 840
   aacatgctgt gtgctggaga cactcggagc ggcgggcccc aggcaaactt gcacgacgcc 900
   tgccagggcg attcgggagg ccccctggtg tgtctgaacg atggccgcat gactttggtg 960
   ggcatcatca gctggggcct gggctgtgga cagaaggatg tcccgggtgt gtacacaaag 1020
   gttaccaact acctagactg gattcgtgac aacatgcgac cgtga                 1065
<210> 8
   <211> 377
   <212> PRT
   <213> Artificial Sequence
<220>
   <223> Description of Artificial Sequence: OmpA-K2S fusion protein
<400> 8
   Met Lys Lys Thr Ala Ile Ala Ile Ala Val Ala Leu Ala Gly Phe Ala
    1               5                  10                  15
   Thr Val Ala Gln Ala Ala Ser Glu Gly Asn Ser Asp Cys Tyr Phe Gly
               20                  25                  30
   Asn Gly Ser Ala Tyr Arg Gly Thr His Ser Leu Thr Glu Ser Gly Ala
           35                  40                  45
   Ser Cys Leu Pro Trp Asn Ser Met Ile Leu Ile Gly Lys Val Tyr Thr
   50 55 60
   Ala Gln Asn Pro Ser Ala Gln Ala Leu Gly Leu Gly Lys His Asn Tyr
   65 70 75 80
   Cys Arg Asn Pro Asp Gly Asp Ala Lys Pro Trp Cys His Val Leu Lys
   85 90 95
   Asn Arg Arg Leu Thr Trp Glu Tyr Cys Asp Val Pro Ser Cys Ser Thr
   100 105 110
   Cys Gly Leu Arg Gln Tyr Ser Gln Pro Gln Phe Arg Ile Lys Gly Gly
   115 120 125
   Leu Phe Ala Asp Ile Ala Ser His Pro Trp Gln Ala Ala Ile Phe Ala
   130 135 140
   Lys His Arg Arg Ser Pro Gly Glu Arg Phe Leu Cys Gly Gly Ile Leu
   145 150 155 160
   Ile Ser Ser Cys Trp Ile Leu Ser Ala Ala His Cys Phe Gln Glu Arg
   165 170 175
   Phe Pro Pro His His Leu Thr Val Ile Leu Gly Arg Thr Tyr Arg Val
   180 185 190
   Val Pro Gly Glu Glu Glu Gln Lys Phe Glu Val Glu Lys Tyr Ile Val
   195 200 205
   His Lys Glu Phe Asp Asp Asp Thr Tyr Asp Asn Asp Ile Ala Leu Leu
   210 215 220
   Gln Leu Lys Ser Asp Ser Ser Arg Cys Ala Gln Glu Ser Ser Val Val
   225 230 235 240
   Arg Thr Val Cys Leu Pro Pro Ala Asp Leu Gln Leu Pro Asp Trp Thr
   245 250 255
   Glu Cys Glu Leu Ser Gly Tyr Gly Lys His Glu Ala Leu Ser Pro Phe
   260 265 270
   Tyr Ser Glu Arg Leu Lys Glu Ala His Val Arg Leu Tyr Pro Ser Ser
   275 280 285
   Arg Cys Thr Ser Gln His Leu Leu Asn Arg Thr Val Thr Asp Asn Met
   290 295 300
   Leu Cys Ala Gly Asp Thr Arg Ser Gly Gly Pro Gln Ala Asn Leu His
   305 310 315 320
   Asp Ala Cys Gln Gly Asp Ser Gly Gly Pro Leu Val Cys Leu Asn Asp
   325 330 335
   Gly Arg Met Thr Leu Val Gly Ile Ile Ser Trp Gly Leu Gly Cys Gly
   340 345 350
   Gln Lys Asp Val Pro Gly Val Tyr Thr Lys Val Thr Asn Tyr Leu Asp
   355 360 365
   Trp Ile Arg Asp Asn Met Arg Pro Gly
   370 375
<210> 9
   <211> 4
   <212> PRT
   <213> Artificial Sequence
<220>
   <223> Description of Artificial Sequence: peptide sequence
<400> 9
   Ser Glu Gly Asn
   1
<210> 10
   <211> 6
   <212> PRT
   <213> Artificial Sequence
<220>
   <223> Description of Artificial Sequence: peptide sequence
<400> 10
   Ser Glu Gly Asn Ser Asp
    1               5
<210> 11
   <211> 354
   <212> PRT
   <213> Artificial Sequence
<220>
   <223> Description of Artificial Sequence: K2S 174-527
<400> 11
   Ser Glu Gly Asn Ser Asp Cys Tyr Phe Gly Asn Gly Ser Ala Tyr Arg
    1               5                  10                  15
   Gly Thr His Ser Leu Thr Glu Ser Gly Ala Ser Cys Leu Pro Trp Asn
               20                  25                  30
   Ser Met Ile Leu Ile Gly Lys Val Tyr Thr Ala Gln Asn Pro Ser Ala
           35                  40                  45
   Gln Ala Leu Gly Leu Gly Lys His Asn Tyr Cys Arg Asn Pro Asp Gly
       50                  55                  60
   Asp Ala Lys Pro Trp Cys His Val Leu Lys Asn Arg Arg Leu Thr Trp
   65                  70                  75                  80
   Glu Tyr Cys Asp Val Pro Ser Cys Ser Thr Cys Gly Leu Arg Gln Tyr
                       85                  90                  95
   Ser Gln Pro Gln Phe Arg Ile Lys Gly Gly Leu Phe Ala Asp Ile Ala
               100                 105                 110
   Ser His Pro Trp Gln Ala Ala Ile Phe Ala Lys His Arg Arg Ser Pro
           115                 120                 125
   Gly Glu Arg Phe Leu Cys Gly Gly Ile Leu Ile Ser Ser Cys Trp Ile
       130                 135                 140
   Leu Ser Ala Ala His Cys Phe Gln Glu Arg Phe Pro Pro His His Leu
   145                 150                 155                 160
   Thr Val Ile Leu Gly Arg Thr Tyr Arg Val Val Pro Gly Glu Glu Glu
                   165                 170                 175
   Gln Lys Phe Glu Val Glu Lys Tyr Ile Val His Lys Glu Phe Asp Asp
               180                 185                 190
   Asp Thr Tyr Asp Asn Asp Ile Ala Leu Leu Gln Leu Lys Ser Asp Ser
           195                 200                 205
   Ser Arg Cys Ala Gln Glu Ser Ser Val Val Arg Thr Val Cys Leu Pro
       210                 215                 220
   Pro Ala Asp Leu Gln Leu Pro Asp Trp Thr Glu Cys Glu Leu Ser Gly
   225                 230                 235                 240
   Tyr Gly Lys His Glu Ala Leu Ser Pro Phe Tyr Ser Glu Arg Leu Lys
                   245                 250                 255
   Glu Ala His Val Arg Leu Tyr Pro Ser Ser Arg Cys Thr Ser Gln His
               260                 265                 270
   Leu Leu Asn Arg Thr Val Thr Asp Asn Met Leu Cys Ala Gly Asp Thr
           275                 280                 285
   Arg Ser Gly Gly Pro Gln Ala Asn Leu His Asp Ala Cys Gln Gly Asp
   290 295 300
   Ser Gly Gly Pro Leu Val Cys Leu Asn Asp Gly Arg Met Thr Leu Val
   305 310 315 320
   Gly Ile Ile Ser Trp Gly Leu Gly Cys Gly Gln Lys Asp Val Pro Gly
   325 330 335
   Val Tyr Thr Lys Val Thr Asn Tyr Leu Asp Trp Ile Arg Asp Asn Met
   340 345 350
   Arg Pro
<210> 12
   <211> 331
   <212> PRT
   <213> Artificial Sequence
<220>
   <223> Description of Artificial Sequence: K2S 197-527
<400> 12
   Ser Gly Ala Ser Cys Leu Pro Trp Asn Ser Met Ile Leu Ile Gly Lys
   1 5 10 15
   Val Tyr Thr Ala Gln Asn Pro Ser Ala Gln Ala Leu Gly Leu Gly Lys
   20 25 30
   His Asn Tyr Cys Arg Asn Pro Asp Gly Asp Ala Lys Pro Trp Cys His
   35 40 45
   Val Leu Lys Asn Arg Arg Leu Thr Trp Glu Tyr Cys Asp Val Pro Ser
   50 55 60
   Cys Ser Thr Cys Gly Leu Arg Gln Tyr Ser Gln Pro Gln Phe Arg Ile
   65 70 75 80
   Lys Gly Gly Leu Phe Ala Asp Ile Ala Ser His Pro Trp Gln Ala Ala
   85 90 95
   Ile Phe Ala Lys His Arg Arg Ser Pro Gly Glu Arg Phe Leu Cys Gly
   100 105 110
   Gly Ile Leu Ile Ser Ser Cys Trp Ile Leu Ser Ala Ala His Cys Phe
   115 120 125
   Gln Glu Arg Phe Pro Pro His His Leu Thr Val Ile Leu Gly Arg Thr
   130 135 140
   Tyr Arg Val Val Pro Gly Glu Glu Glu Gln Lys Phe Glu Val Glu Lys
   145 150 155 160
   Tyr Ile Val His Lys Glu Phe Asp Asp Asp Thr Tyr Asp Asn Asp Ile
   165 170 175
   Ala Leu Leu Gln Leu Lys Ser Asp Ser Ser Arg Cys Ala Gln Glu Ser
   180 185 190
   Ser Val Val Arg Thr Val Cys Leu Pro Pro Ala Asp Leu Gln Leu Pro
   195 200 205
   Asp Trp Thr Glu Cys Glu Leu Ser Gly Tyr Gly Lys His Glu Ala Leu
   210 215 220
   Ser Pro Phe Tyr Ser Glu Arg Leu Lys Glu Ala His Val Arg Leu Tyr
   225 230 235 240
   Pro Ser Ser Arg Cys Thr Ser Gln His Leu Leu Asn Arg Thr Val Thr
   245 250 255
   Asp Asn Met Leu Cys Ala Gly Asp Thr Arg Ser Gly Gly Pro Gln Ala
   260 265 270
   Asn Leu His Asp Ala Cys Gln Gly Asp Ser Gly Gly Pro Leu Val Cys
   275 280 285
   Leu Asn Asp Gly Arg Met Thr Leu Val Gly Ile Ile Ser Trp Gly Leu
   290 295 300
   Gly Cys Gly Gln Lys Asp Val Pro Gly Val Tyr Thr Lys Val Thr Asn
   305 310 315 320
   Tyr Leu Asp Trp Ile Arg Asp Asn Met Arg Pro
   325 330
<210> 13
   <211> 339
   <212> PRT
   <213> Artificial Sequence
<220>
   <223> Description of Artificial Sequence: K2S 193-527, modified
<400> 13
   Ser Glu Gly Asn Ser Leu Thr Glu Ser Gly Ala Ser Cys Leu Pro Trp
   1 5 10 15
   Asn Ser Met Ile Leu Ile Gly Lys Val Tyr Thr Ala Gln Asn Pro Ser
   20 25 30
   Ala Gln Ala Leu Gly Leu Gly Lys His Asn Tyr Cys Arg Asn Pro Asp
   35 40 45
   Gly Asp Ala Lys Pro Trp Cys His Val Leu Lys Asn Arg Arg Leu Thr
   50 55 60
   Trp Glu Tyr Cys Asp Val Pro Ser Cys Ser Thr Cys Gly Leu Arg Gln
   65 70 75 80
   Tyr Ser Gln Pro Gln Phe Arg Ile Lys Gly Gly Leu Phe Ala Asp Ile
   85 90 95
   Ala Ser His Pro Trp Gln Ala Ala Ile Phe Ala Lys His Arg Arg Ser
                   100                 105                 110
   Pro Gly Glu Arg Phe Leu Cys Gly Gly Ile Leu Ile Ser Ser Cys Trp
           115                 120                 125
   Ile Leu Ser Ala Ala His Cys Phe Gln Glu Arg Phe Pro Pro His His
       130                 135                 140
   Leu Thr Val Ile Leu Gly Arg Thr Tyr Arg Val Val Pro Gly Glu Glu
   145                 150                 155                 160
   Glu Gln Lys Phe Glu Val Glu Lys Tyr Ile Val His Lys Glu Phe Asp
                   165                 170                 175
   Asp Asp Thr Tyr Asp Asn Asp Ile Ala Leu Leu Gln Leu Lys Ser Asp
               180                 185                 190
   Ser Ser Arg Cys Ala Gln Glu Ser Ser Val Val Arg Thr Val Cys Leu
           195                 200                 205
   Pro Pro Ala Asp Leu Gln Leu Pro Asp Trp Thr Glu Cys Glu Leu Ser
       210                 215                 220
   Gly Tyr Gly Lys His Glu Ala Leu Ser Pro Phe Tyr Ser Glu Arg Leu
   225                 230                 235                 240
   Lys Glu Ala His Val Arg Leu Tyr Pro Ser Ser Arg Cys Thr Ser Gln
                   245                 250                 255
   His Leu Leu Asn Arg Thr Val Thr Asp Asn Met Leu Cys Ala Gly Asp
               260                 265                 270
   Thr Arg Ser Gly Gly Pro Gln Ala Asn Leu His Asp Ala Cys Gln Gly
           275                 280                 285
   Asp Ser Gly Gly Pro Leu Val Cys Leu Asn Asp Gly Arg Met Thr Leu
       290                 295                 300
   Val Gly Ile Ile Ser Trp Gly Leu Gly Cys Gly Gln Lys Asp Val Pro
   305 310 315 320
   Gly Val Tyr Thr Lys Val Thr Asn Tyr Leu Asp Trp Ile Arg Asp Asn
   325 330 335
   Met Arg Pro
<210> 14
   <211> 335
   <212> PRT
   <213> Artificial Sequence
<220>
   <223> Description of Artificial Sequence: K2S 193-527, modified
<400> 14
   Ser Leu Thr Glu Ser Gly Ala Ser Cys Leu Pro Trp Asn Ser Met Ile
   1 5 10 15
   Leu Ile Gly Lys Val Tyr Thr Ala Gln Asn Pro Ser Ala Gln Ala Leu
   20 25 30
   Gly Leu Gly Lys His Asn Tyr Cys Arg Asn Pro Asp Gly Asp Ala Lys
   35 40 45
   Pro Trp Cys His Val Leu Lys Asn Arg Arg Leu Thr Trp Glu Tyr Cys
   50 55 60
   Asp Val Pro Ser Ser Ser Thr Cys Gly Leu Arg Gln Tyr Ser Gln Pro
   65 70 75 80
   Gln Phe Arg Ile Lys Gly Gly Leu Phe Ala Asp Ile Ala Ser His Pro
   85 90 95
   Trp Gln Ala Ala Ile Phe Ala Lys His Arg Arg Ser Pro Gly Glu Arg
   100 105 110
   Phe Leu Cys Gly Gly Ile Leu Ile Ser Ser Cys Trp Ile Leu Ser Ala
   115 120 125
   Ala His Cys Phe Gln Glu Arg Phe Pro Pro His His Leu Thr Val Ile
   130 135 140
   Leu Gly Arg Thr Tyr Arg Val Val Pro Gly Glu Glu Glu Gln Lys Phe
   145 150 155 160
   Glu Val Glu Lys Tyr Ile Val His Lys Glu Phe Asp Asp Asp Thr Tyr
   165 170 175
   Asp Asn Asp Ile Ala Leu Leu Gln Leu Lys Ser Asp Ser Ser Arg Cys
   180 185 190
   Ala Gln Glu Ser Ser Val Val Arg Thr Val Cys Leu Pro Pro Ala Asp
   195 200 205
   Leu Gln Leu Pro Asp Trp Thr Glu Cys Glu Leu Ser Gly Tyr Gly Lys
   210 215 220
   His Glu Ala Leu Ser Pro Phe Tyr Ser Glu Arg Leu Lys Glu Ala His
   225 230 235 240
   Val Arg Leu Tyr Pro Ser Ser Arg Cys Thr Ser Gln His Leu Leu Asn
   245 250 255
   Arg Thr Val Thr Asp Asn Met Leu Cys Ala Gly Asp Thr Arg Ser Gly
   260 265 270
   Gly Pro Gln Ala Asn Leu His Asp Ala Cys Gln Gly Asp Ser Gly Gly
   275 280 285
   Pro Leu Val Cys Leu Asn Asp Gly Arg Met Thr Leu Val Gly Ile Ile
   290 295 300
   Ser Trp Gly Leu Gly Cys Gly Gln Lys Asp Val Pro Gly Val Tyr Thr
   305 310 315 320
   Lys Val Thr Asn Tyr Leu Asp Trp Ile Arg Asp Asn Met Arg Pro
   325 330 335
<210> 15
   <211> 343
   <212> PRT
   <213> Artificial Sequence
<220>
   <223> Description of Artificial Sequence: K2S 191-527, modified
<400> 15
   Ser Glu Gly Asn Ser Asp Thr His Ser Leu Thr Glu Ser Gly Ala Ser
   1 5 10 15
   Cys Leu Pro Trp Asn Ser Met Ile Leu Ile Gly Lys Val Tyr Thr Ala
   20 25 30
   Gln Asn Pro Ser Ala Gln Ala Leu Gly Leu Gly Lys His Asn Tyr Cys
   35 40 45
   Arg Asn Pro Asp Gly Asp Ala Lys Pro Trp Cys His Val Leu Lys Asn
   50 55 60
   Arg Arg Leu Thr Trp Glu Tyr Cys Asp Val Pro Ser Cys Ser Thr Cys
   65 70 75 80
   Gly Leu Arg Gln Tyr Ser Gln Pro Gln Phe Arg Ile Lys Gly Gly Leu
   85 90 95
   Phe Ala Asp Ile Ala Ser His Pro Trp Gln Ala Ala Ile Phe Ala Lys
   100 105 110
   His Arg Arg Ser Pro Gly Glu Arg Phe Leu Cys Gly Gly Ile Leu Ile
   115 120 125
   Ser Ser Cys Trp Ile Leu Ser Ala Ala His Cys Phe Gln Glu Arg Phe
       130                 135                 140
   Pro Pro His His Leu Thr Val Ile Leu Gly Arg Thr Tyr Arg Val Val
   145                 150                 155                 160
   Pro Gly Glu Glu Glu Gln Lys Phe Glu Val Glu Lys Tyr Ile Val His
                   165                 170                 175
   Lys Glu Phe Asp Asp Asp Thr Tyr Asp Asn Asp Ile Ala Leu Leu Gln
               180                 185                 190
   Leu Lys Ser Asp Ser Ser Arg Cys Ala Gln Glu Ser Ser Val Val Arg
           195                 200                 205
   Thr Val Cys Leu Pro Pro Ala Asp Leu Gln Leu Pro Asp Trp Thr Glu
       210                 215                 220
   Cys Glu Leu Ser Gly Tyr Gly Lys His Glu Ala Leu Ser Pro Phe Tyr
   225                 230                 235                 240
   Ser Glu Arg Leu Lys Glu Ala His Val Arg Leu Tyr Pro Ser Ser Arg
                   245                 250                 255
   Cys Thr Ser Gln His Leu Leu Asn Arg Thr Val Thr Asp Asn Met Leu
               260                 265                 270
   Cys Ala Gly Asp Thr Arg Ser Gly Gly Pro Gln Ala Asn Leu His Asp
           275                 280                 285
   Ala Cys Gln Gly Asp Ser Gly Gly Pro Leu Val Cys Leu Asn Asp Gly
       290                 295                 300
   Arg Met Thr Leu Val Gly Ile Ile Ser Trp Gly Leu Gly Cys Gly Gln
   305                 310                 315                 320
   Lys Asp Val Pro Gly Val Tyr Thr Lys Val Thr Asn Tyr Leu Asp Trp
                   325                 330                 335
   Ile Arg Asp Asn Met Arg Pro
   340
<210> 16
   <211> 343
   <212> PRT
   <213> Artificial Sequence
<220>
   <223> Description of Artificial Sequence: K2S 191-527, modified
<400> 16
   Ser Glu Gly Asn Ser Asp Thr His Ser Leu Thr Glu Ser Gly Ala Ser
   1 5 10 15
   Cys Leu Pro Trp Asn Ser Met Ile Leu Ile Gly Lys Val Tyr Thr Ala
   20 25 30
   Gln Asn Pro Ser Ala Gln Ala Leu Gly Leu Gly Lys His Asn Tyr Cys
   35 40 45
   Arg Asn Pro Asp Gly Asp Ala Lys Pro Trp Cys His Val Leu Lys Asn
   50 55 60
   Arg Arg Leu Thr Trp Glu Tyr Cys Asp Val Pro Ser Ser Ser Thr Cys
   65 70 75 80
   Gly Leu Arg Gln Tyr Ser Gln Pro Gln Phe Arg Ile Lys Gly Gly Leu
   85 90 95
   Phe Ala Asp Ile Ala Ser His Pro Trp Gln Ala Ala Ile Phe Ala Lys
   100 105 110
   His Arg Arg Ser Pro Gly Glu Arg Phe Leu Cys Gly Gly Ile Leu Ile
   115 120 125
   Ser Ser Cys Trp Ile Leu Ser Ala Ala His Cys Phe Gln Glu Arg Phe
   130 135 140
   Pro Pro His His Leu Thr Val Ile Leu Gly Arg Thr Tyr Arg Val Val
   145 150 155 160
   Pro Gly Glu Glu Glu Gln Lys Phe Glu Val Glu Lys Tyr Ile Val His
   165 170 175
   Lys Glu Phe Asp Asp Asp Thr Tyr Asp Asn Asp Ile Ala Leu Leu Gln
   180 185 190
   Leu Lys Ser Asp Ser Ser Arg Cys Ala Gln Glu Ser Ser Val Val Arg
   195 200 205
   Thr Val Cys Leu Pro Pro Ala Asp Leu Gln Leu Pro Asp Trp Thr Glu
   210 215 220
   Cys Glu Leu Ser Gly Tyr Gly Lys His Glu Ala Leu Ser Pro Phe Tyr
   225 230 235 240
   Ser Glu Arg Leu Lys Glu Ala His Val Arg Leu Tyr Pro Ser Ser Arg
   245 250 255
   Cys Thr Ser Gln His Leu Leu Asn Arg Thr Val Thr Asp Asn Met Leu
   260 265 270
   Cys Ala Gly Asp Thr Arg Ser Gly Gly Pro Gln Ala Asn Leu His Asp
   275 280 285
   Ala Cys Gln Gly Asp Ser Gly Gly Pro Leu Val Cys Leu Asn Asp Gly
   290 295 300
   Arg Met Thr Leu Val Gly Ile Ile Ser Trp Gly Leu Gly Cys Gly Gln
   305 310 315 320
   Lys Asp Val Pro Gly Val Tyr Thr Lys Val Thr Asn Tyr Leu Asp Trp
   325 330 335
   Ile Arg Asp Asn Met Arg Pro
   340
<210> 17
   <211> 308
   <212> PRT
   <213> Artificial Sequence
<220>
   <223> Description of Artificial Sequence: K2S 220-527
<400> 17
   Ser Ala Gln Ala Leu Gly Leu Gly Lys His Asn Tyr Cys Arg Asn Pro
   1               5                   10                  15
   Asp Gly Asp Ala Lys Pro Trp Cys His Val Leu Lys Asn Arg Arg Leu
               20                  25                  30
   Thr Trp Glu Tyr Cys Asp Val Pro Ser Cys Ser Thr Cys Gly Leu Arg
           35                  40                  45
   Gln Tyr Ser Gln Pro Gln Phe Arg Ile Lys Gly Gly Leu Phe Ala Asp
       50                  55                  60
   Ile Ala Ser His Pro Trp Gln Ala Ala Ile Phe Ala Lys His Arg Arg
   65                  70                  75                  80
   Ser Pro Gly Glu Arg Phe Leu Cys Gly Gly Ile Leu Ile Ser Ser Cys
                   85                  90                  95
   Trp Ile Leu Ser Ala Ala His Cys Phe Gln Glu Arg Phe Pro Pro His
               100                 105                 110
   His Leu Thr Val Ile Leu Gly Arg Thr Tyr Arg Val Val Pro Gly Glu
           115                 120                 125
   Glu Glu Gln Lys Phe Glu Val Glu Lys Tyr Ile Val His Lys Glu Phe
       130                 135                 140
   Asp Asp Asp Thr Tyr Asp Asn Asp Ile Ala Leu Leu Gln Leu Lys Ser
   145 150 155 160
   Asp Ser Ser Arg Cys Ala Gln Glu Ser Ser Val Val Arg Thr Val Cys
   165 170 175
   Leu Pro Pro Ala Asp Leu Gln Leu Pro Asp Trp Thr Glu Cys Glu Leu
   180 185 190
   Ser Gly Tyr Gly Lys His Glu Ala Leu Ser Pro Phe Tyr Ser Glu Arg
   195 200 205
   Leu Lys Glu Ala His Val Arg Leu Tyr Pro Ser Ser Arg Cys Thr Ser
   210 215 220
   Gln His Leu Leu Asn Arg Thr Val Thr Asp Asn Met Leu Cys Ala Gly
   225 230 235 240
   Asp Thr Arg Ser Gly Gly Pro Gln Ala Asn Leu His Asp Ala Cys Gln
   245 250 255
   Gly Asp Ser Gly Gly Pro Leu Val Cys Leu Asn Asp Gly Arg Met Thr
   260 265 270
   Leu Val Gly Ile Ile Ser Trp Gly Leu Gly Cys Gly Gln Lys Asp Val
   275 280 285
   Pro Gly Val Tyr Thr Lys Val Thr Asn Tyr Leu Asp Trp Ile Arg Asp
   290 295 300
   Asn Met Arg Pro
   305
<210> 18
   <211> 268
   <212> PRT
   <213> Artificial Sequence
<220>
   <223> Description of Artificial Sequence: K2S 260-527
<400> 18
   Ser Cys Ser Thr Cys Gly Leu Arg Gln Tyr Ser Gln Pro Gln Phe Arg
    1               5                  10                  15
   Ile Lys Gly Gly Leu Phe Ala Asp Ile Ala Ser His Pro Trp Gln Ala
               20                  25                  30
   Ala Ile Phe Ala Lys His Arg Arg Ser Pro Gly Glu Arg Phe Leu Cys
           35                  40                  45
   Gly Gly Ile Leu Ile Ser Ser Cys Trp Ile Leu Ser Ala Ala His Cys
       50                  55                  60
   Phe Gln Glu Arg Phe Pro Pro His His Leu Thr Val Ile Leu Gly Arg
   65                  70                  75                  80
   Thr Tyr Arg Val Val Pro Gly Glu Glu Glu Gln Lys Phe Glu Val Glu
                   85                  90                  95
   Lys Tyr Ile Val His Lys Glu Phe Asp Asp Asp Thr Tyr Asp Asn Asp
               100                 105                 110
   Ile Ala Leu Leu Gln Leu Lys Ser Asp Ser Ser Arg Cys Ala Gln Glu
           115                 120                 125
   Ser Ser Val Val Arg Thr Val Cys Leu Pro Pro Ala Asp Leu Gln Leu
       130                 135                 140
   Pro Asp Trp Thr Glu Cys Glu Leu Ser Gly Tyr Gly Lys His Glu Ala
   145                 150                 155                 160
   Leu Ser Pro Phe Tyr Ser Glu Arg Leu Lys Glu Ala His Val Arg Leu
                   165                 170                 175
   Tyr Pro Ser Ser Arg Cys Thr Ser Gln His Leu Leu Asn Arg Thr Val
               180                 185                 190
   Thr Asp Asn Met Leu Cys Ala Gly Asp Thr Arg Ser Gly Gly Pro Gln
           195                 200                 205
   Ala Asn Leu His Asp Ala Cys Gln Gly Asp Ser Gly Gly Pro Leu Val
       210                 215                 220
   Cys Leu Asn Asp Gly Arg Met Thr Leu Val Gly Ile Ile Ser Trp Gly
   225                 230                 235                 240
   Leu Gly Cys Gly Gln Lys Asp Val Pro Gly Val Tyr Thr Lys Val Thr
                   245                 250                 255
   Asn Tyr Leu Asp Trp Ile Arg Asp Asn Met Arg Pro
               260                 265
<210> 19
   <211> 527
   <212> PRT
   <213> Homo sapiens
<400> 19
   Ser Tyr Gln Val Ile Cys Arg Asp Glu Lys Thr Gln Met Ile Tyr Gln
   1               5                   10                  15
   Gln His Gln Ser Trp Leu Arg Pro Val Leu Arg Ser Asn Arg Val Glu
               20                  25                  30
   Tyr Cys Trp Cys Asn Ser Gly Arg Ala Gln Cys His Ser Val Pro Val
           35                  40                  45
   Lys Ser Cys Ser Glu Pro Arg Cys Phe Asn Gly Gly Thr Cys Gln Gln
       50                  55                  60
   Ala Leu Tyr Phe Ser Asp Phe Val Cys Gln Cys Pro Glu Gly Phe Ala
   65                  70                  75                  80
   Gly Lys Cys Cys Glu Ile Asp Thr Arg Ala Thr Cys Tyr Glu Asp Gln
   85 90 95
   Gly Ile Ser Tyr Arg Gly Thr Trp Ser Thr Ala Glu Ser Gly Ala Glu
   100 105 110
   Cys Thr Asn Trp Asn Ser Ser Ala Leu Ala Gln Lys Pro Tyr Ser Gly
   115 120 125
   Arg Arg Pro Asp Ala Ile Arg Leu Gly Leu Gly Asn His Asn Tyr Cys
   130 135 140
   Arg Asn Pro Asp Arg Asp Ser Lys Pro Trp Cys Tyr Val Phe Lys Ala
   145 150 155 160
   Gly Lys Tyr Ser Ser Glu Phe Cys Ser Thr Pro Ala Cys Ser Glu Gly
   165 170 175
   Asn Ser Asp Cys Tyr Phe Gly Asn Gly Ser Ala Tyr Arg Gly Thr His
   180 185 190
   Ser Leu Thr Glu Ser Gly Ala Ser Cys Leu Pro Trp Asn Ser Met Ile
   195 200 205
   Leu Ile Gly Lys Val Tyr Thr Ala Gln Asn Pro Ser Ala Gln Ala Leu
   210 215 220
   Gly Leu Gly Lys His Asn Tyr Cys Arg Asn Pro Asp Gly Asp Ala Lys
   225 230 235 240
   Pro Trp Cys His Val Leu Lys Asn Arg Arg Leu Thr Trp Glu Tyr Cys
   245 250 255
   Asp Val Pro Ser Cys Ser Thr Cys Gly Leu Arg Gln Tyr Ser Gln Pro
   260 265 270
   Gln Phe Arg Ile Lys Gly Gly Leu Phe Ala Asp Ile Ala Ser His Pro
   275 280 285
   Trp Gln Ala Ala Ile Phe Ala Lys His Arg Arg Ser Pro Gly Glu Arg
   290 295 300
   Phe Leu Cys Gly Gly Ile Leu Ile Ser Ser Cys Trp Ile Leu Ser Ala
   305 310 315 320
   Ala His Cys Phe Gln Glu Arg Phe Pro Pro His His Leu Thr Val Ile
   325 330 335
   Leu Gly Arg Thr Tyr Arg Val Val Pro Gly Glu Glu Glu Gln Lys Phe
   340 345 350
   Glu Val Glu Lys Tyr Ile Val His Lys Glu Phe Asp Asp Asp Thr Tyr
   355 360 365
   Asp Asn Asp Ile Ala Leu Leu Gln Leu Lys Ser Asp Ser Ser Arg Cys
   370 375 380
   Ala Gln Glu Ser Ser Val Val Arg Thr Val Cys Leu Pro Pro Ala Asp
   385 390 395 400
   Leu Gln Leu Pro Asp Trp Thr Glu Cys Glu Leu Ser Gly Tyr Gly Lys
   405 410 415
   His Glu Ala Leu Ser Pro Phe Tyr Ser Glu Arg Leu Lys Glu Ala His
   420 425 430
   Val Arg Leu Tyr Pro Ser Ser Arg Cys Thr Ser Gln His Leu Leu Asn
   435 440 445
   Arg Thr Val Thr Asp Asn Met Leu Cys Ala Gly Asp Thr Arg Ser Gly
   450 455 460
   Gly Pro Gln Ala Asn Leu His Asp Ala Cys Gln Gly Asp Ser Gly Gly
   465 470 475 480
   Pro Leu Val Cys Leu Asn Asp Gly Arg Met Thr Leu Val Gly Ile Ile
   485 490 495
   Ser Trp Gly Leu Gly Cys Gly Gln Lys Asp Val Pro Gly Val Tyr Thr
   500 505 510
   Lys Val Thr Asn Tyr Leu Asp Trp Ile Arg Asp Asn Met Arg Pro
   515 520 525
<210> 20
   <211> 12
   <212> DNA
   <213> Artificial Sequence
<220>
   <223> Description of Artificial Sequence: coding sequence for SEGN
<400> 20
   tctgagggaa ac 12
<210> 21
   <211> 22
   <212> PRT
   <213> Escherichia coli
<400> 21
   Met Lys Lys Thr Ala Ile Ala Ile Ala Val Ala Leu Ala Gly Phe Ala
   1 5 10 15
   Thr Val Ala Gln Ala Ala
   20
<210> 22
   <211> 42
   <212> DNA
   <213> Artificial Sequence
<220>
   <223> Description of Artificial Sequence: primer
<400> 22
   gaggaggagg tggcccaggc ggcctctgag ggaaacagtg ac 42
<210> 23
   <211> 42
   <212> DNA
   <213> Artificial Sequence
<220>
   <223> Description of Artificial Sequence: primer
<400> 23
   gaggaggagc tggccggcct ggcccggtcg catgttgtca cg 42
<210> 24
   <211> 26
   <212> DNA
   <213> Artificial Sequence
<220>
   <223> Description of Artificial Sequence: primer
<400> 24
   acatgcgacc gtgacaggcc ggccag 26
<210> 25
   <211> 26
   <212> DNA
   <213> Artificial Sequence
<220>
   <223> Description of Artificial Sequence: primer
<400> 25
   ctggccggcc tgtcacggtc gcatgt 26

## Description

The invention belongs to the field of thrombolysis and of tissue plasminogen activator (tPA) derivative production in prokaryotic cells.

The invention relates to methods for the production of a recombinant DNA-derived tPA, a variant thereof or a (Kringle 2 Serine) K2S molecule or a variant thereof in prokaryotic cells, wherein said tPA or K2S or variant is secreted extracellularly as an active and correctly folded protein, and the prokaryotic cell contains and expresses a vector comprising the DNA coding for said tPA or K2S or variant operably linked to the DNA coding for the signal peptide OmpA. The invention further relates to specific K2S derivatives obtainable by said method. The invention further relates to said DNA molecules and the use of said DNA molecules in said methods.

### Background art

Tissue plasminogen activator (tPA) is a polypeptide containing 527 amino acid residues (27) with a molecular mass of 72 kDa. The molecule is divided into five structural domains. Nearby the N-terminal region is a looped finger domain, which is followed by a growth factor domain. Two similar domains, kringle 1 and kringle 2, are following. Both finger and kringle 2 domains bind specifically to the fibrin clots thereby accelerating tPA protein activation of bound plasminogen. Downstream of kringle 2 is the serine protease, with its catalytic site located at the C-terminus. The serine protease is responsible for converting plasminogen to plasmin a reaction important in the homeostasis of fibrin formation and clot dissolution. The correct folding of tPA requires the correct pairing of 17 disulfide bridges in the molecule (1).

Clinically, tPA is a thrombolytic agent of choice for the treatment of acute myocardial infarction, pulmonary embolism, stroke, peripheral arterial occlusions, and other thromboembolic diseases. It has the advantage of causing no side effects on systemic haemorrhaging and fibrinogen depletion (7). Bowes melanoma cells were first used as a source in tPA production for therapeutic purposes (12). Since a consistent process with efficient production of highly purified protein in good yield is required for clinical use, the construction of full-length recombinant-tPA (r-tPA) progressed to mammalian cells. Chinese hamster ovary cells were transfected with the tPA gene to synthesize the r-tPA (8, 22). The recombinant DNA derived product produced by a mammalian cell culture fermentation system is harvested and purified from the culture medium. Attracted by simplicity and economy of production, a number of efforts in producing r-tPA from microorganisms, expecially bacteria, and more especially from *Escherichia coli,* were investigated (10, 13, 30). Regarding the low yield and the formation of inclusion bodies, which resulted in misfolding and in an inactive enzyme, numerous strategies have been proposed to overcome these problems.

Several deletion-mutant variants including kringle 2 plus serine protease *(K2S)* were considered. However, the enzymatic activity of the recombinant-K2S (r-K2S) was obtained only when refolding processes of purified inclusion bodies from cytoplasmic compartment were achieved (16, 29). In order to avoid the cumbersome refolding processes, impurities of misfolded proteins, and periplasmic protein delivery, special bacterial expression systems were exploited (6, 31). Despite periplasmic expression of tPA, overexpression led to inactive aggregates, even in the relatively high oxidizing condition in the periplasm.

In the prior art, there are a few descriptions of methods for the preparation of recombinant K2S in E. coli. However, there is no disclosure of a method leading to a cost effective method for large scale production of biologically active K2S.

Obukowicz et al. (25) expressed and purified r-K2S from periplasmic space. In addition, EP 1 048 732 describes expression and purification of recombinant tPA as PelB-fusion protein. The obvious disadvantage of the methods provided by Obukowicz et al and EP 1 048 732 was an extra periplasmic extraction step, which is not suitable for large scale production.Moreover, the production yield obtained with the PelB-K2S fusion protein is very low (about 4 µg/l).

Saito et al. (29) disclose the cytoplasmic expression of r-K2S. The authors used an in vivo renaturation processes for the expressed r-K2S, which was purified from the cytoplasmic space of E. coli as inclusion body. Boehringer Mannheim use a similar cumbersome denaturing/refolding process involving the steps of cell digestion, solubilization under denaturing and reducing conditions and reactivation under oxidizing conditions in the presence of GSH/GSSG which is not cost effective (24) and requires mutation of the amino acid sequence with possibly antigenic potential.

In EP 0 302 456 cytoplasmatic expression of several tPA variants in E.coli is described. The tPA variants were purified from the cruide cell extract after cell lysis via benzamidine Sepharose and a monoclonal antibody affinity chromatography step.

In 1991, Waldenström et al. (34) constructed a vector (pEZZK2P) for the secretion of kringle 2 plus serine protease domain to E. coli culture supernatant. Hydroxylamine was used to remove the ZZ fusion peptide from IgG-Sepharose purified fraction. The cleavage agent hydroxylamine required modification of the cleavage sites of kringle 2 plus serine protease (Asn¹⁷⁷ → Ser and Asn¹⁸⁴ → Gln) thus to protect it from hydroxylamine digestion. However, the resulting non-native, not properly folded K2S molecule is not suitable for therapeutic purposes. No enzymatic activity regarding fibrin binding/protease activity was disclosed. The unusual sequence may even activate the human immune system.

WO 98/54199 provides a method of producing and purifying a tissue-plasminogen activator-like protease (tPALP), a homolog of the tPA. The tPALPs are expressed in fusion with a Histidin-tag in E.coli and purified from the cytoplasma after denaturation. Beyond the purification step the tPALPs have to be refolded.

The problem underlying the present invention was thus to provide a commercially applicable method for large scale production of tPA molecules and derivatives thereof, e.g. K2S, wherein the K2S molecule is secreted in its biologically active form into the culture supernatant.

### Description of the invention

The problem was solved within the scope of the claims and specification of the present invention.

The use of the singular or plural in the claims or specification is in no way intended to be limiting and also includes the other form.

The invention relates to a method for the production of a recombinant DNA-derived tissue plasminogen activator (tPA), a tPA variant, a Kringle 2 Serine protease molecule (K2S) or a K2S variant in prokaryotic cells, wherein said tPA, tPA variant, K2S molecule or K2S variant is secreted extracellularly as an active and correctly folded protein, characterized in that the prokaryotic cell contains and expresses a vector comprising the DNA coding for said tPA, tPA variant, K2S molecule or K2S variant operably linked to the DNA coding for the signal peptide OmpA.

Surprisingly, the use of the signal peptide OmpA alone and/ or in combination with the N-terminal amino acids SEGN (SEQ ID NO:9) / SEGNSD (SEQ ID NO:10) translocate the recombinant DNA-derived tPA, tPA variant, K2S molecule or K2S variant to the outer surface and facilitates the release of the functional and active molecule into the culture medium to a greater extent than any other method in the prior art. Before crossing the outer membrane, the recombinant DNA-derived protein is correctly folded according to the method of the present invention. The signal peptide is cleaved off to produce a mature molecule. Surprisingly, the efficiency of signal peptide removal is very high and leads to correct folding of the recombinant DNA-derived protein.

Said signal peptide OmpA interacts with SecE and is delivered across the inner membrane by energy generated by SecA, which binds to Sec components (SecE-SecY). SecY forms a secretion pore to dispatch the recombinant DNA-derived protein according to the invention. The space between the outer membrane and inner membrane of Gram-negative bacteria, periplasm, has higher oxidative condition in comparison to the cytoplasmic space. This supports the formation of disulfide bonds and properly folding of the recombinant DNA-derived protein (e.g. K2S) in the periplasm to yield an active molecule. According to the present invention, the signal peptide will be cleaved off to produce a mature molecule. The complex of GspD secretin and GspS lipoprotein on the outer membrane serves as gate channel for secreting the recombinant DNA-derived protein according to the invention to the extracellular medium. This secretion process requires energy, which is generated in cytoplasm by GspE nucleotide-binding protein then transferred to the inner membrane protein (Gsp G-J, F and K-N). GspC transfers the energy to GspD by forming a cross-linker between a set of inner membrane protein (Gsp G-J, F and K-N) and GspD. Before crossing the outer membrane successfully, the recombinant DNA-derived protein is correctly folded.

Operably linked according to the invention means that the DNA encoding the tPA, tPA variant, K2S molecule or K2S variant (preferably comprising the nucleic acid encoding SEGN or SEGNSD at its N-terminal portion) is cloned in close proximity to the OmpA DNA into the vector in order to achieve expression of the OmpA-tPA, tPA variant, K2S molecule or K2S variant-fusion protein and to direct secretion outside the prokaryotic host cell. Typically, the majority of the tPA, tPA variant, K2S molecule or K2S variant is secreted and can then be purified by appropriate methods such as ammonium sulfate precipitation and/or affinity chromatography and further purification steps. The invention also includes the use of inducers such as IPTG or IPTG in combination with glycerol, the improvement of the incubation condition and harvesting period to maximize the amount of active protein.

In a preferred embodiment, said DNA encoding the OmpA signal peptide may be fused to a short peptide characterized by the amino acid sequence SEGN or SEGNSD or the coding nucleic acid sequence TCTGAGGGAAAC (SEQ ID NO:20) or TCTGAGGGAAACAGTGAC (SEQ ID NO:1) and located in the N-terminal portion or at the N-terminal portion of the tPA, tPA variant, K2S molecule or K2S variant. Thus, preferably, said fusion protein comprises OmpA-SEGNSD-tPA, -tPA-variant, -K2S-molecule or -K2S-variant. Even more preferred, said amino acids characterized by SEGN or SEGNSD may be carry a point mutation or may be substituted by a non-natural amino acid. Even more preferred, there may be an amino acid or non-amino acid spacer between OmpA and SEGN or SEGNSD and the tPA, tPA variant, K2S molecule or K2S variant.

Thus, in a preferred method according to the invention said the prokaryotic cell contains and expresses a vector comprising the DNA coding for said tPA, tPA variant, K2S molecule or K2S variant operably linked to the DNA coding for the signal peptide OmpA, which is operably linked to the nucleic acid molecule defined by the sequence TCTGAGGGAAACAGTGAC or a functional derivative thereof.

The method according to the invention comprises prokaryotic host cells such as, but not limited to *Escherichia coli (E. coli), Bacillus subtilis, Streptomyces, Pseudomonas, e.g. Pseudomonas putida, Proteus mirabilis, Saccharomyces, Pichia* or *Staphylococcus,* e.g. *Staphylococcus carnosus.* Preferably said host cells according to the invention are Gram-negative bacteria.

Preferably, a method according to the invention is also characterised in that the prokaryotic cell is *E. coli.* Suitable strains include, but are not limited to E. coli XL-1 blue, BL21(DE3), JM109, DH series, TOP10 and HB101.

Preferably, a method according to the invention is also characterised in that the following steps are carried out:
a) the DNA encoding the tPA, tPA variant, K2S molecule or K2S variant is amplified by PCR;
b) the PCR product is purified;
c) said PCR product is inserted into a vector comprising the DNA coding for OmpA signal peptide and the DNA coding for gpIII in such a way that said PCR product is operably linked upstream to the DNA coding for the OmpA signal sequence and linked downstream to the DNA coding for gpIII of said vector;
d) that a stop codon is inserted between said tPA, tPA variant, K2S molecule or K2S variant and gpIII;
e) said vector is expressed by the prokaryotic cell
f) the tPA, tPA variant, K2S molecule or K2S variant is purified.

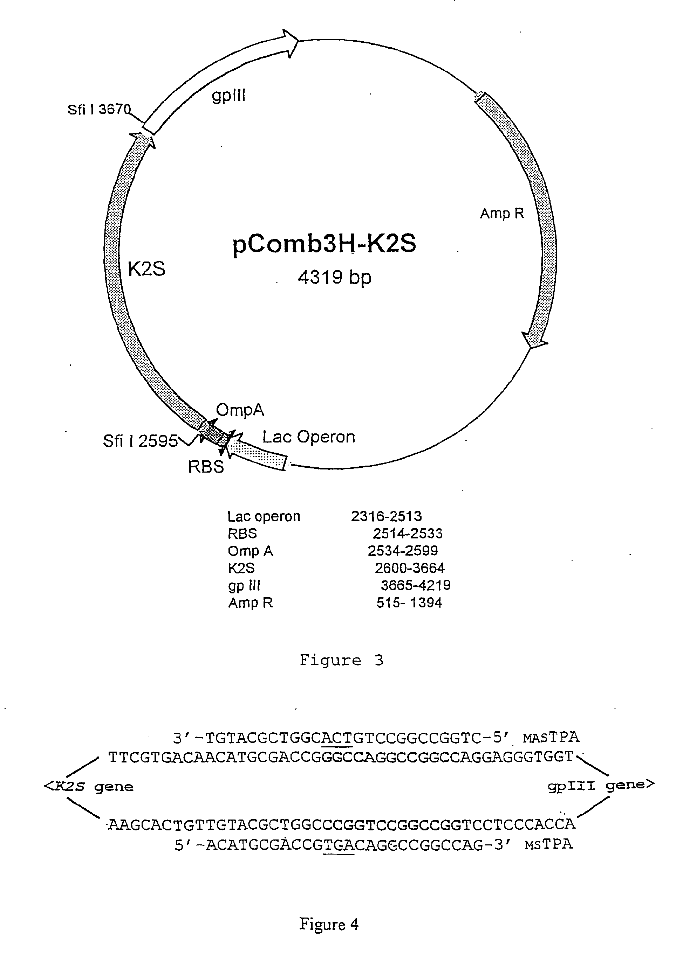

For step a) according to the invention the choice / design of the primers is important to clone the DNA in the right location and direction of the expression vector (see example 1). Thus, the primers as exemplified in example 1 and figure 4 comprise an important aspect of the present invention. With gp III of step c) gene protein III is meant which is present mainly in phagemid vectors. The stop codon is inserted to avoid transcription of gp III thus eventually leading to secretion of the tPA, tPA variant, K2S molecule or K2S variant of interest. Any suitable method for insertion of the stop codon may be employed such as site-directed mutagenesis (e.g. Weiner MP, Costa GL (1994) PCR Methods Appl 4(3):S131-136; Weiner MP, Costa GL, Schoettlin W, Cline J, Mathur E, Bauer JC (1994) Gene 151(1-2):119-123; see also example 1).

Any vector may be used in the method according to the invention, preferably said vector is a phagemid vector (see below).

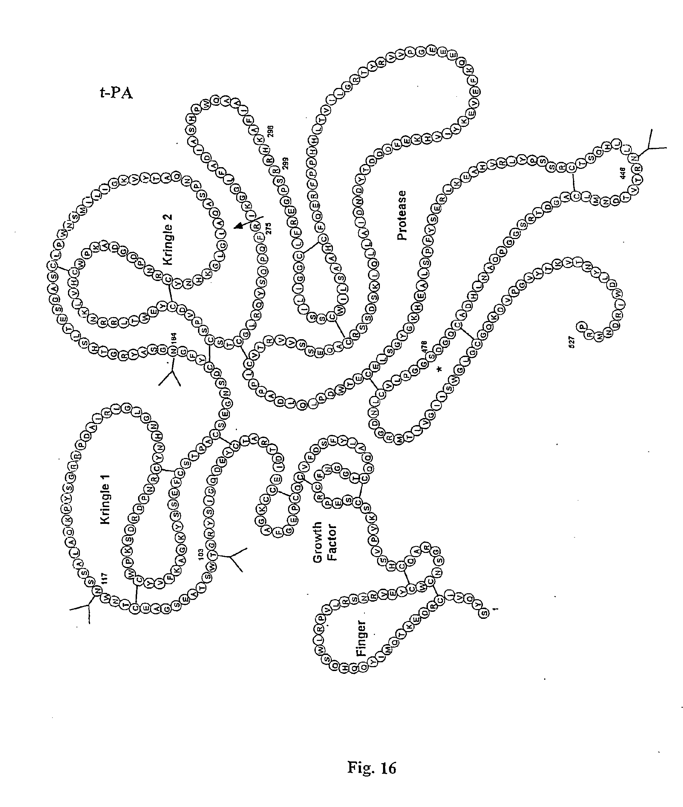

Preferably, a method according to the invention is also characterised in that the tPA, tPA variant, K2S molecule or K2S variant is selected from human tissue plasminogen activator (tPA, figure 16) or a fragment, a functional variant, an allelic variant, a subunit, a chemical derivative, a fusion protein or a glycosylation variant thereof. Such fragments, allelic variants, functional variants, variants based on the degenerative nucleic acid code, fusion proteins with an tPA protein according to the invention, chemical derivatives or a glycosylation variant of the tPA proteins according to the invention may include one, several or all of the following domains or subunits or variants thereof:
1. Finger domain (4-50)
2. Growth factor domain (50-87)
3. Kringle 1 domain (87-176)
4. Kringle 2 domain (176-262)
5. Protease domain (276-527)

The numbering/naming of the domains is according to Genbank accession number GI 137119 or Nature 301 (5897), 214-221 (1983).

More preferably, a method according to the invention is also characterised in that the tPA, tPA variant, K2S molecule or K2S variant is selected from the Kringle 2 (4.) plus Serine protease (5.) K2S variant of human tissue plasminogen activator or a fragment, a functional variant, an allelic variant, a subunit, a chemical derivative, a fusion protein or a glycosylation variant thereof. More preferably, a method according to the invention is also characterised in that the vector is a phagemid vector comprising the DNA coding for OmpA signal peptide and the DNA coding for gpIII.

More preferably, a method according to the invention is also characterised in that the vector is the pComb3HSS phagemid (see also example 1).

More preferably, a method according to the invention is also characterised in that the DNA sequence comprises or consists of the following DNA sequence encoding OmpA, and K2S or a functional variant of K2S or a variant due to the degenerate nucleotide code:

More preferably, a method according to the invention is also characterised in that the DNA Sequence of OmpA consists of the following sequence:

Said DNA encodes the following amino acid sequence of OmpA. OmpA thus consists of a protein characterized by the following amino acid sequence or a glycosylation variant thereof as part of the invention:
MKKTAIAIAVALAGFATVAQAA (SEQ ID NO:21).

The untranslated region may contain a regulatory element, such as e.g. a transcription initiation unit (promoter) or enhancer. Said promoter may, for example, be a constitutive, inducible or development-controlled promoter. Preferably, without ruling out other known promoters, the constitutive promoters of the human Cytomegalovirus (CMV) and Rous sarcoma virus (RSV), as well as the Simian virus 40 (SV40) and Herpes simplex promoter. Inducible promoters according to the invention comprise antibiotic-resistant promoters, heat-shock promoters, hormone-inducible "Mammary tumour virus promoter" and the metallothioneine promoter. Preferred promoters include T3 promoter, T7 promoter, Lac/aral and Ltet0-1.

More preferably, a method according to the invention is also characterised in that the DNA of the tPA, tPA variant, K2S molecule or K2S variant is preceded by a lac promoter and/or a ribosomal binding site such as the Shine-Dalgarno sequence (see also example).

More preferably, a method according to the invention is also characterised in that the DNA coding for the tPA, tPA variant, K2S molecule or K2S variant is selected from the group of DNA molecules coding for at least 90% of the amino acids 87 - 527, 174 - 527, 180 - 527 or 220 - 527 of the human tissue plasminogen activator protein.

More preferably, a method according to the invention is also characterised in that the DNA Sequence of K2S comprises or consists of the following sequence:

The present invention also relates to variants of the before-mentioned nucleic acid molecules due to the degenerate code or to fragments thereof, nucleic acids which hybridize to said nucleic acids under stringent conditions, allelic or functional variants. The invention also relates to nucleic acids comprising said K2S nucleic acid fused to the nucleic acid encoding another protein molecule.

Stringent conditions as understood by the skilled person are conditions which select for more than 85 %, preferred more than 90 % homology (Sambrook et al. 1989; Molecular Cloning: A Laboratory Manual, 2nd ed., Cold Spring Harbor Laboratory Press, Cold Spring Harbor, New York). The hybridisation will be carried out e.g. in 6x SSC/ 5x Denhardt's solution/ 0,1 % SDS (SDS: sodium dodecylsulfate) at 65 °C. The degree of stringency is decided in the washing step. Thus, for example for a selection of DNA-sequences with approx. 85 % or more homology, the conditions 0,2 x SSC/ 0,01 % SDS/ 65 °C and for a selection of DNA-sequences of approx. 90 % or more homology the conditions 0,1x SSC/ 0,01 % SDS/ 65 °C are suitable. The composition of said reagents is described in Sambrook et al. (1989, supra).

Another important part of the present invention is a variant of human tissue plasminogen activator comprising of or consisting of the Kringle 2 (4.) plus Serine protease (5.) (abbreviated K2S) protein or a variant or a fragment, a functional variant, an allelic variant, a subunit, a chemical derivative, a fusion protein or a glycosylation variant thereof.

The numbering/naming of the domains is according to Genbank accession number GI 137119 or Nature 301 (5897), 214-221 (1983), wherein the Kringle 2 domain extends from amino acid 176-262 and the protease domain from 276-527. Thus, according to the invention, a preferred K2S molecule may include amino acids 176-527 including the amino acids between Kringle 2 and the protease (amino acids 263 to 275; exemplified in fig. (structure A)). A K2S molecule according to the invention comprises the minimal part of the Kringle 2 domain and the protease domain still retaining protease activity and fibrin binding activity (measured as exemplified in the description/example). Said K2S molecule according to the invention comprises the amino acids SEGN or SEGNSD in its N-terminal portion (see infra). A preferred K2S molecule does not include amino acids 1 to 3 or 1 to 5 of the tPA molecule. Preferably, a K2S molecule according to the invention has the amino acid Asn at positions 177 and 184, i.e. it does not require the modifications as disclosed in Waldenström for improved producibility with a method according to the invention. Thus, a preferred K2S molecule according to the invention has the native amino acid sequence (no mutation) as opposed to the molecules known from the prior art. Most preferred, said K2S molecule according to the invention is a molecule characterized by the native amino acid sequence or parts thereof, does neither have amino acids 1 to 3 nor 1 to 5 of tPA and comprises N-terminally the amino acids SEGN or SEGNSD for improved producibility and/or correct folding of the molecule.

It is essential that the K2S protein according to the invention comprises in its N-terminal portion a peptide characterized by the amino acid sequence SEGN which advantageously allows commercial production with a method as described supra leading to a correctly folded, secreted K2S protein. Said 4 amino acids characterized by SEGN may have one or several amino acids more N-terminal, however said amino acids have to be located in the N-terminal portion as opposed to the C-terminal portion. Most preferably, said amino acids are located at the N-terminal portion. Preferably, the amino acids characterized by SEGN may be carry a point mutation or may be substituted by a non-natural amino acid.

Thus, in another important embodiment the invention relates to a K2S protein characterized in that it comprises the amino acids defined by the sequence SEGN or a variant or a fragment, a functional variant, an allelic variant, a subunit, a chemical derivative, a fusion protein or a glycosylation variant thereof.

Such fragments are exemplified e.g. in figure 10 (Structure B-1) and figure 11 (Structure B-2) extending from amino acids 193-527. Structure B-1 has the native amino acid Cys in position 261, wherein in B-2 the amino acid is substituted by Ser. Further fragments according to the invention comprising the amino acids 220-527 (fig. 14, structure C) or comprising the amino acids 260-527 (fig. 15, structure D) may be modified according to the invention by addition of the amino acids SEGN and/or substitution of Cys-261 by Ser. The artisan can determine the minimal length of a K2S molecule according to the invention in order to retain its biological function and generate a K2S molecule with improved producibility and/or correct folding by adding the amino acids SEGN in the N-terminal portion. Thus, another preferred embodiment is said minimal K2S molecule with SEGN at its N-terminal portion.

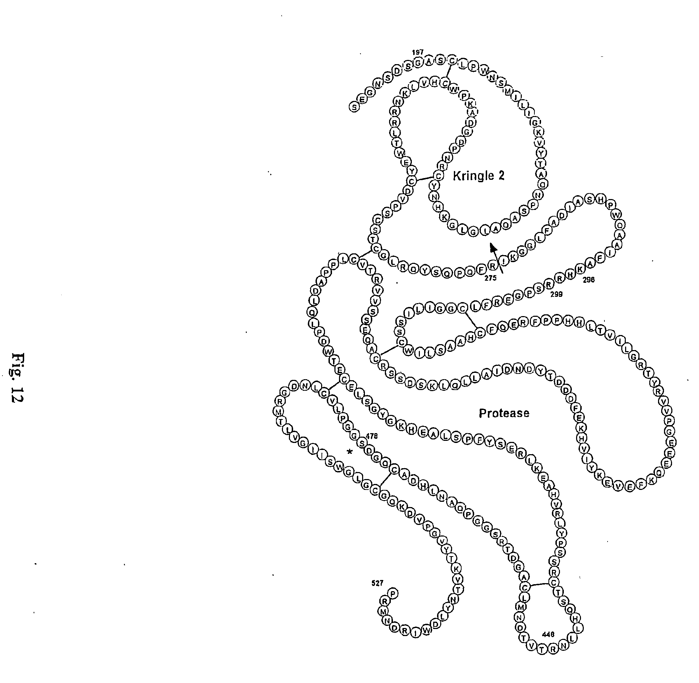

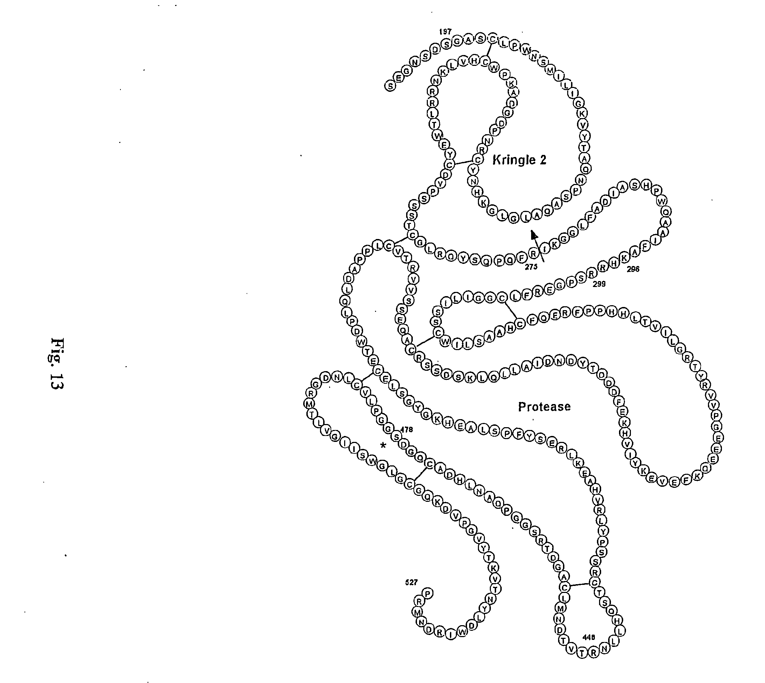

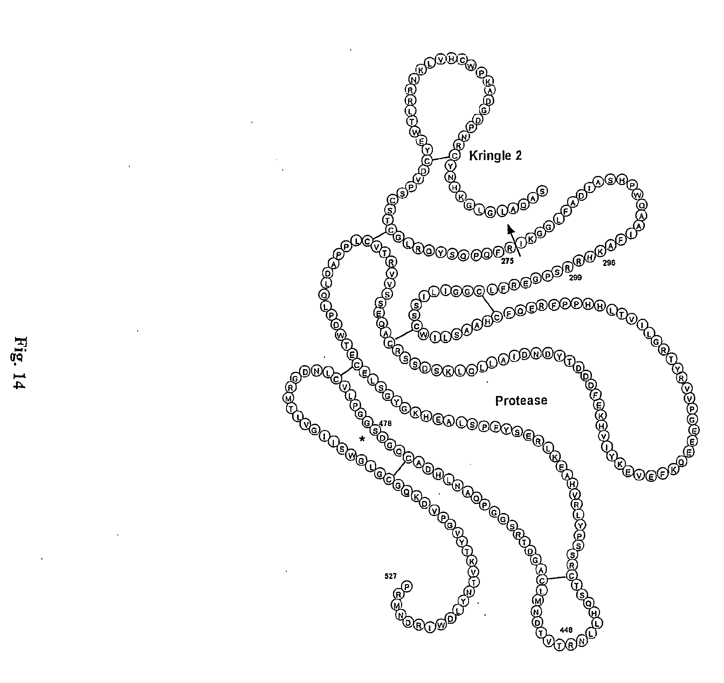

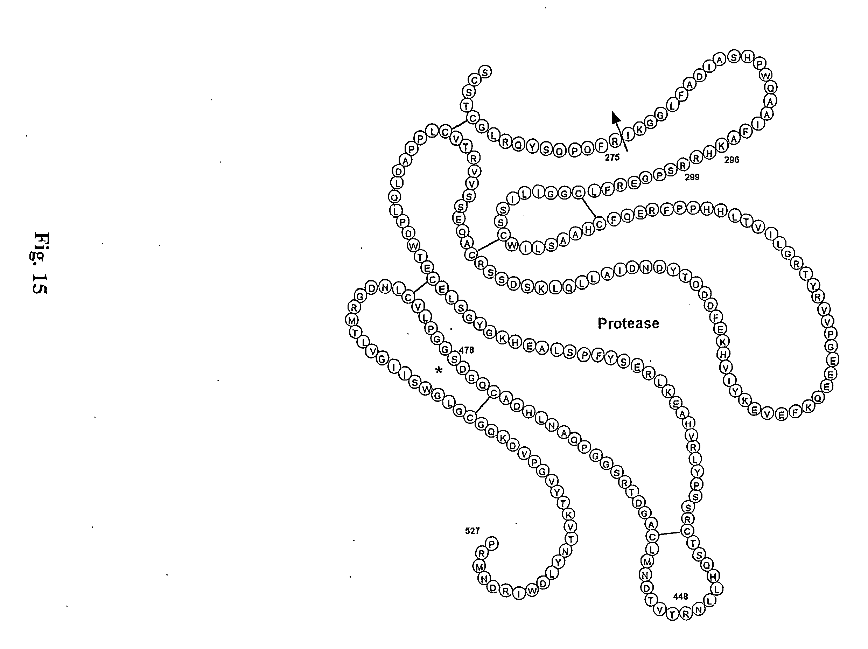

In another important embodiment the invention relates to a K2S protein characterized in that it comprises the amino acids defined by the sequence SEGNSD or a variant or a fragment, a functional variant, an allelic variant, a subunit, a chemical derivative, a fusion protein or a glycosylation variant thereof. Such fragments are exemplified e.g. in figure 12 (Structure B-3) and figure 13 (Structure B-4) extending from amino acids 191-527. Structure B-3 has the native amino acid Cys in position 261, wherein in B-4 the amino acid is substituted by Ser. Further fragments according to the invention comprising the amino acids 220-527 (fig. 14, structure C) or comprising the amino acids 260-527 (fig. 15, structure D) may be modified according to the invention by addition of the amino acids SEGNSD and/or substitution of Cys-261 by Ser. The artisan can determine the minimal length of a K2S molecule according to the invention in order to retain its biological function and generate a K2S molecule with improved producibility and/or correct folding by adding the amino acids SEGNSD in the N-terminal portion. Thus, another preferred embodiment is said minimal K2S molecule with SEGNSD at its N-terminal portion.

The application furthermore discloses a K2S protein comprising a protein characterized by the following amino acid sequence or a variant or a fragment, a functional variant, an allelic variant, a subunit, a chemical derivative or a glycosylation variant thereof:

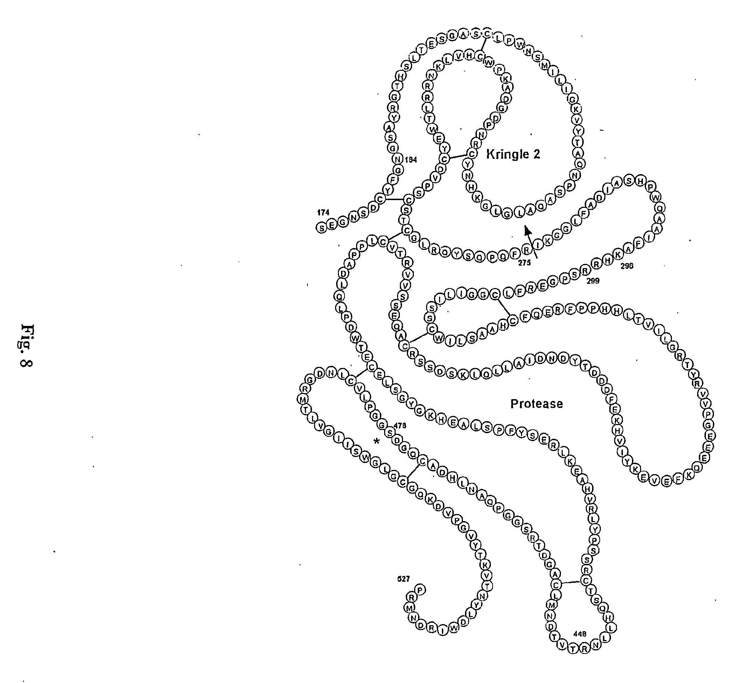

According to the invention, * means STOP (i.e. encoded by a stop codon). This K2S molecule is exemplified in figure 8.

One variant of the K2S molecule according to the invention relates to a fusion protein of K2S being fused to another protein molecule.

The application furthermore discloses a K2S protein consisting of a protein characterized by the following amino acid sequence:

Said K2S molecules may be encoded by a DNA molecule as described supra.

Another important aspect of the invention relates to a DNA molecule characterized in that it is coding for:
a) the OmpA protein operably linked to
b) a DNA molecule coding for a polypeptide containing the kringle 2 domain and the serine protease domain of tissue plasminogen activator protein.

More preferably, a DNA molecule according to the invention is also characterised in that the DNA sequence comprises or consists of the following DNA sequence encoding OmpA, and K2S or a functional variant of K2S or a variant due to the degenerate nucleotide code:

Said DNA molecule encodes the following fusion protein of OmpA and K2S. Said fusion protein of OmpA, and K2S characterised in that it comprises or consists of a protein characterized by the following amino acid sequence or a fragment, a functional variant, an allelic variant, a subunit, a chemical derivative or a glycosylation variant thereof forms an important part of the present invention:

Another preferred aspect of the invention relates to a DNA molecule according to the invention, characterized in that said DNA sequence b) is coding for at least 90% of the amino acids 87 - 527 of the human tissue plasminogen activator protein (numbering used herein as GI 137119 or Nature 301 (5897), 214-221 (1983).

Another preferred aspect of the invention relates to a DNA molecule according to the invention, characterized in that said DNA sequence b) is coding for at least 90% of the amino acids 174 - 527 of the human tissue plasminogen activator protein.

Another preferred aspect of the invention relates to a DNA molecule according to the invention, characterized in that said DNA sequence b) is coding for at least 90% of the amino acids 180 - 527 of the human tissue plasminogen activator protein.

Another preferred aspect of the invention relates to a DNA molecule according to the invention, characterized in that said DNA sequence b) is coding for at least 90% of the amino acids 220 - 527 of the human tissue plasminogen activator protein.

Another preferred aspect of the invention relates to a DNA molecule according to the invention, characterized in that said DNA sequence a) is hybridizing under stringent conditions to the following sequence:

Another preferred aspect of the invention relates to a DNA molecule according to the invention, characterized in that said DNA sequence a) consists of the following sequence:

Another preferred aspect of the invention relates to a DNA molecule according to the invention, characterized in that said DNA sequence b) is hybridizing under stringent conditions to the following sequence:

Another preferred aspect of the invention relates to a DNA molecule according to the invention, characterized in that said DNA sequence b) consists of the following sequence:

Another preferred embodiment of the invention relates to a vector containing a DNA sequence according to the invention.

Another preferred embodiment of the invention relates to a vector according to the invention, wherein said DNA sequence is preceded by a lac promoter and a ribosomal binding site. Suitable vectors according to the invention include, but are not limited to viral vectors such as e.g. Vaccinia, Semliki-Forest-Virus and Adenovirus, phagemid vectors and the like. Preferred are vectors which can be advantageously used in E. coli, but also in any other prokaryotic host such as pPROTet.E, pPROLar.A, members of the pBAD family, pSE family, pQE family and pCAL. Another preferred embodiment of the invention relates to the vector pComb3HSS containing a DNA according to the invention, wherein the expression of the gp III protein is suppressed or inhibited by deleting the DNA molecule encoding said gp III protein or by a stop codon between the gene coding for a polypeptide containing the kringle 2 domain and the serine protease domain of tissue plasminogen activator protein and the protein III gene.

Another important aspect of the present invention relates to a prokaryotic host cell comprising a DNA molecule according to the invention.

Another important aspect of the present invention relates to a prokaryotic host cell comprising a vector according to the invention.

Another important aspect of the present invention relates to an E. coli host cell comprising a DNA molecule according to the invention.

Another important aspect of the present invention relates to a E. coli host cell comprising a vector according to the invention.

Yet another important aspect of the present invention is the use of a DNA molecule according to the invention or of a vector according to the invention or a host cell according to the invention in a method for the production of a polypeptide having the activity of tissue plasminogen activator. Yet another important aspect of the present invention is the use according the invention as described above, wherein said method is a method according to the invention.

Another very important aspect is a pharmaceutical composition comprising a substance obtainable by a method according to the invention and pharmaceutically acceptable excipients and carriers. An example for said substance is the K2S molecule described supra. The term "pharmaceutically acceptable carrier" as used herein refers to conventional pharmaceutic excipients or additives used in the pharmaceutical manufacturing art. Such physiologically acceptable compounds include, for example, carbohydrates, such as glucose, sucrose or dextrans, antioxidants, such as ascorbic acid or glutathione, chelating agents, low molecular weight proteins or other stabilizers or excipients (see also e.g. Remington's Pharmaceutical Sciences (1990, 18th ed. Mack Publ., Easton.)). Said pharmaceutical composition according to the invention can be advantageously administered intravenously as a bolus, e.g. as a single bolus for 5 to 10 seconds intravenously.

The invention further relates to the use of substances obtainable by a method according to the invention in the manufacture of a medicament in the treatment of stroke, cardiac infarction, acute myocardial infarction, pulmonary embolism, any artery occlusion such as coronary artery occlusion, intracranial artery occlusion (e.g. arteries supplying the brain), peripherally occluded arteries, deep vein thrombosis or related diseases associated with unwanted blood clotting.

The following example is intended to aid the understanding of the invention and should in no way be regarded as limiting the scope of the invention.

### Example 1

### MATERIALS AND METHODS

Primer design. In order to amplify a specific part of tPA gene, a pair of primers SK2/174 [5' GAGGAGGAGGTGGCCCAGGCGGCCTCTGAGGGAAACAGTGAC 3'] (SEQ ID NO:22) and ASSP [5'GAGGAGGAGCTGGCCGGCCTGGCCCGGTCGCATGTTGTCACG 3'] (SEQ ID NO:23) were synthesized (Life Technologies, Grand Island, NY). These primers were designed based on the human tPA gene retrieved from NCBI databases (g137119). They were synthesized with Sfi I end cloning sites (underlined) in such a way that the reading frame from the ATG of the gpIII gene in phagemid vector, pComb3HSS, will be maintained throughout the inserted sequence.

Another primer set for site-directed mutagenesis was designed to anneal at the sequence situated between K2S gene and gene III in pComb3H-K2S. The sequence of primers with mutation bases (underlined) for generating a new stop codon were MSTPA [5'ACATGCGACCGTGACAGGCCGGCCAG3'] (SEQ ID NO:24) and MASTPA [5'CTGGCCGGCCTGTCACGGTCGCATGT3'] (SEQ ID NO:25).

Amplification of K2S gene by PCR. One µg SK2/174 and ASSP primers together with 50 ng of p51-3 template (obtained from Dr. Hiroshi Sasaki, Fujisawa Pharmaceutical, Japan) were suspended in 100 µl PCR mixture. An amount of 2.5 U Taq polymerase (Roche Molecular Biochemicals, Indianapolis, IN) was finally added to the solution. The titrated amplification condition was initiated with jump start at 85°C for 4 min, then denaturation at 95°C for 50 sec, annealing at 42°C for 50 sec, extension at 72°C for 1.5 min. Thirty five rounds were repeatedly performed. The mixture was further incubated at 72°C for 10 min. The amplified product of 1110 bp was subsequently purified by QIAquick PCR Purification Kit (QIAGEN, Hilden, Germany). The correctness of purified product was confirmed by restriction enzymes.

Construction of phagemid expressing K2S. The purified PCR product of K2S and pComb3HSS phagemid (kindly provided by Dr. Carlos F. Barbas, Scripps Institute, USA) were digested with Sfi I (Roche Molecular Biochemicals, Indianapolis, IN) to prepare specific cohesive cloning sites. Four µg of the purified PCR product was digested with 60 U of Sfi I at 50°C for 18 h. For pComb3HSS, 20 µg of phagemid vectors were treated with 100 U of Sfi I. Digested products of purified PCR product of K2S and pComb3HSS (∼3300 bp) were subsequently gel-purified by the QIAquick Gel Extraction Kit (QIAGEN, Hilden, Germany). T4 ligase (Roche Molecular Biochemicals, Indianapolis, IN) of 5 U were introduced to the mixture of 0.7 µg of purified Sfi I-digested pComb3HSS and 0.9 µg of purified Sfi I-digested PCR product. Ligation reaction was incubated at 30°C for 18 h. The newly constructed phagemid was named pComb3H-K2S.

Transformation of E. coli XL-1 Blue. Two hundred µl of CaCl₂ competent E. coli XL-1 Blue (Stratagene, La Jolla, CA) were transformed with 70 ng of ligated or mutated product. The transformed cells were propagated by spreading on LB agar containing 100 µg/ml ampicillin and 10 µg/ml tetracycline (Sigma, Saint Louis, MO). After cultivation at 37°C for 18 h several antibiotic resistant colonies were selected for plasmid minipreps by using the alkaline lysis method. Each purified plasmid was subjected to Sfi I restriction site analysis. A transformant harboring plasmid with the correct Sfi I restriction site(s) was subsequently propagated for 18 h at 37°C in 100 ml LB broth with ampicillin 100 µg/ml and tetracycline 10 µg/ml. A plasmid maxiprep was performed using the QIAGEN Plasmid Maxi Kit (QIAGEN, Hilden, Germany). The purified plasmid was reexamined for specific restriction sites by Sfi I and sequenced by AmpliTaq DNA Polymerase Terminator Cycle Sequencing Kit (The Perkin-Elmer Corporation, Forster City, CA).

Site-directed mutagenesis of pComb3H-K2S. 10 ng of pComb3H-K2S template were mixed with 125 ng of MSTPA and MASTPA primers. PfuTurbo DNA polymerase (Stratagene, LA Jolla, CA) of 2.5 U was added to the mixture for cycle amplification. The reaction started with one round of 95°C for 30 sec. Then it was followed by 16 rounds consisting of 95°C for 30 sec, 55°C for 1 min, and 68°C for 9 min. The reaction tube was subsequently placed on ice for 2 min. In order to destroy the template strands, 10 U of Dpn I restriction enzyme (Stratagene, LA Jolla, CA) were added to the amplification reaction and incubated for 1 h at 37°C. This synthesized product (MpComb3H-K2S) was further used to transform E. coli XL-1 Blue.

Preparation of phage-display recombinant-K2S. After pComb3H-K2S was transformed to XL-1 Blue, the phage display technique was performed. A clone of pComb3H-K2S transformed E. coli XL-1 Blue was propagated in 10 ml super broth containing ampicillin 100 µg/ml and tetracycline 10 µg/ml at 37°C until the O.D. [600 nm] of 1.5 was reached. The bacterial culture was subsequently propagated in 100 ml of the same medium and culture for 2 h. An amount of 10¹² pfu of VCSM13 helper phage (Stratagene, La Jolla, CA) was used to infect the transformed E. coli XL-1 Blue. After 3 h incubation, kanamycin at a final concentration of 70 µg/ml final concentration was added to culture. The culture was left shaking (200 RPM) for 18 h at 37°C. Bacteriophages which harbored K2S on gp3 (K2S-ϕ) were then harvested by adding 4% w/v PEG MW 8000 (Sigma, Saint Louis, MO) and 3% w/v NaCl. Finally, the harvested phage was resuspended in 2 ml PBS pH 7.4. The phage number was determined by infecting E. coli XL-1 Blue. The colony-forming unit per milliliter (cfu/ml) was calculated as described previously (21).

Expression of recombinant-K2S in shaker flasks. MpComb3H-K2S transformed E. coli XL-1 Blue was cultivated in 100 ml super broth (3% w/v tryptone, 2% w/v yeast extract and 1% w/v MOPS) at pH 7.0 in the presence of ampicillin (100 µg/ml) at 37°C until an O.D. [600 nm] of 0.8 was reached. Subsequently, the protein synthesis was induced by 1 mM of IPTG (Promega, Madison, WI). The bacteria were further cultured shaking (200 RPM) for 6 h at 30°C. The culture supernatant was collected and precipitated with 55% saturated ammonium sulfate (32). The precipitate was reconstituted with PBS, pH 7.2, and dialysed in the same buffer solution at 4°C for 18 h. Periplasmic proteins from bacterial cells were extracted by using a chloroform shock as previously described by Ames et al. (2).

Immunoassay quantification of recombinant-K2S. In order to detect r-K2S, solid phase was coated with monoclonal anti-kringle 2 domain (16/B) (generously provided by Dr. Ute Zacharias, Central Institute of Molecular Biology, Berlin-Buch, Germany). The standard ELISA washing and blocking processes were preformed. Fifty µl of 10¹¹ cfu/ml of K2S-ϕ secretory r-K2S were added into each anti-kringle 2 coated well. Antigen-antibody detection was carried out as follows. Either sheep anti-M13 conjugated HRP (Pharmacia Biotech, Uppsala, Sweden) or sheep anti-tPA conjugated HRP (Cedarlane, Ontario, Canada), was added to each reaction well after the washing step. The substrate TMB was subjected to every well and the reaction was finally ceased with H₂SO₄ solution after 30 min incubation. The standard melanoma tPA 86/670 (National Institute for Biological Standards and Control, Hertfordshine, UK) was used as positive control.

Amidolytic activity assay. A test kit for the detection of tPA amidolytic activity was purchased from Chromogenix (Molndal, Sweden). The substrate mixture containing plasminogen and S-2251 was used to determine serine protease enzymatic activity. The dilution of 10⁻² of each ammonium precipitated sample was assayed with and without stimulator, human fibrinogen fragments. The assay procedure was according to the COASET t-PA manual.

SDS-PAGE and immunoblotting. The dialysed precipitate-product from culture supernatant was further concentrated 10 folds with centricon 10 (AMICON , Beverly, MA). The concentrated sample was subjected to protein separation by SDS-PAGE, 15% resolving gel, in the reducing buffer followed by electroblotting to nitrocellulose. The nitrocellulose was then blocked with 4% skimmed milk for 2 hr. In order to detect r-K2S, a proper dilution of sheep anti-tPA conjugated HRP was applied to the nitrocellulose. The immunoreactive band was visualized by a sensitive detection system, Amplified Opti-4CN kit (BIORAD, Hercules, CA).

Copolymerized plasminogen polyacrylamide gel electrophoresis. An 11% resolving polyacrylamide gel was copolymerized with plasminogen and gelatin as previously described by Heussen et al. (14). The stacking gel was prepared as 4 % concentration without plasminogen and gelatin. Electrophoresis was performed at 4°C at a constant current of 8 mA. The residual SDS in gel slab was removed after gentle shaking at room temperature for 1h in 2.5% Triton X-100. Then the gel slab was incubated in 0.1 M glycine-NaOH, pH 8.3, for 5 h at 37°C. Finally, the gel slab was stained and destained by standard Coomassie brilliant blue (R-250) dying system. The location of the peptide harboring enzymatic activity was not stained by dye in contrast to blue-paint background.

### RESULTS

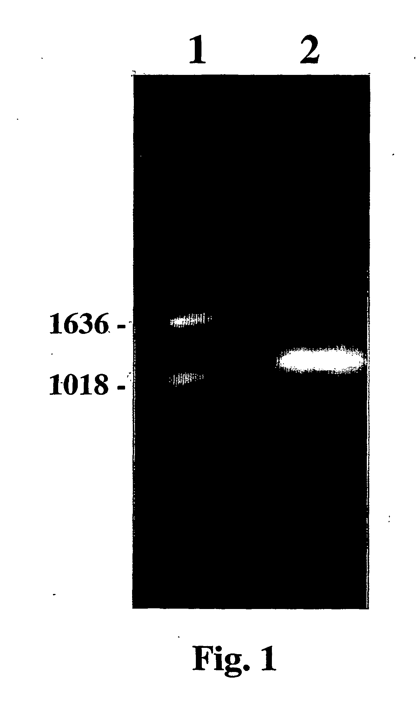

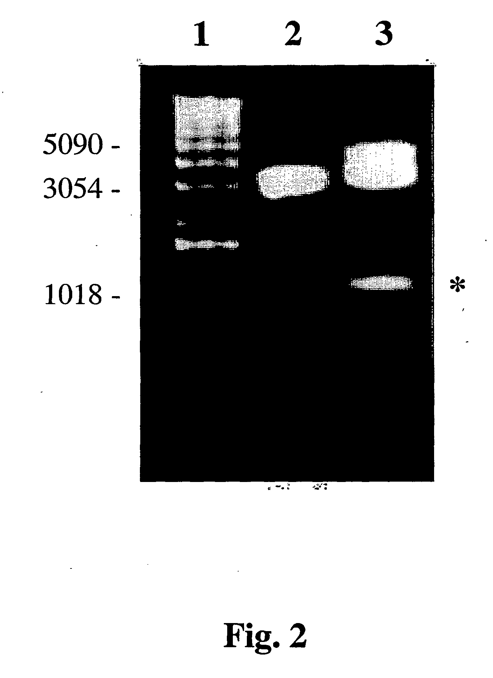

Construction of K2S gene carrying vector. From the vector p51-3 we amplified the kringle 2 plus ther serine protease portion of tPA (Ser¹⁷⁴ in kringle 2 domain to Pro⁵²⁷ in the serine protease) using primers SK2/174 and ASSP. The amplified 1110 bp product was demonstrated by agarose gel electrophoresis (Fig. 1, lane 2) and was inserted into pComb3HSS phagemid by double Sfi I cleavage sites on 5' and 3' ends in the correct reading frame. Thus a new vector, pComb3H-K2S, harboring the K2S was generated. In this vector K2S is flanked upstream by the OmpA signal sequence and donwstream by gp3. The correct insertion of K2S was verified both by restriction analysis with Sfi I (Fig. 2, lane 3), PCR-analysis (demonstration of a single band at 1110 bp), and DNA sequencing. The schematic diagram of pComb3H-K2S map is given in Fig. 3.

Phage-displayed r-K2S. VCSM13 filamentous phage was used to infect pComb3H-K2S transformed E. coli XL-1 Blue, X[K2S]. VCSM13 was propagated and incorporated the K2S-gp3 fusion protein during the viral packaging processes. The harvested recombinant phage (K2S-ϕ) gave a concentration of 5.4 x 10¹¹ cfu/ml determined by reinfecting E. coli XL-1 Blue with PEG-precipitated phages. These recombinant phage particles were verified for the expression of r-K2S by sandwich ELISA. The phage-bound heterologous K2S protein was recognized by the monoclonal anti-kringle 2 antibody (16B) by using sheep anti-tPA conjugated HRP antibody detection system. The absorbance of this assay was 1.12 ± 0.03 (Table 1). The amount of K2S detectable on 10¹² phage particles is equal to 336 ng of protein in relation to the standard melanoma tPA. In order to corroborate that K2S-gp3 fusion protein was associated with phage particles, sheep anti-tPA conjugated HRP antibody was substituted by sheep anti-M13 antibody conjugated HRP. This immuno-reaction exhibited an absorbance of 1.89 ± 0.07 (Table 1). In contrast, if the capture antibody was sheep anti-M13 antibody, extremely low K2S was observed with sheep anti-tPA antibody conjugated HRP; the absorbance was only 0.17 ± 0.01 (Table 1). This suggested that only a minority of purified phage particles carried K2S-gp3 fusion protein. VCSM13 prepared from non-transformed XL-1 Blue was used as a negative control.

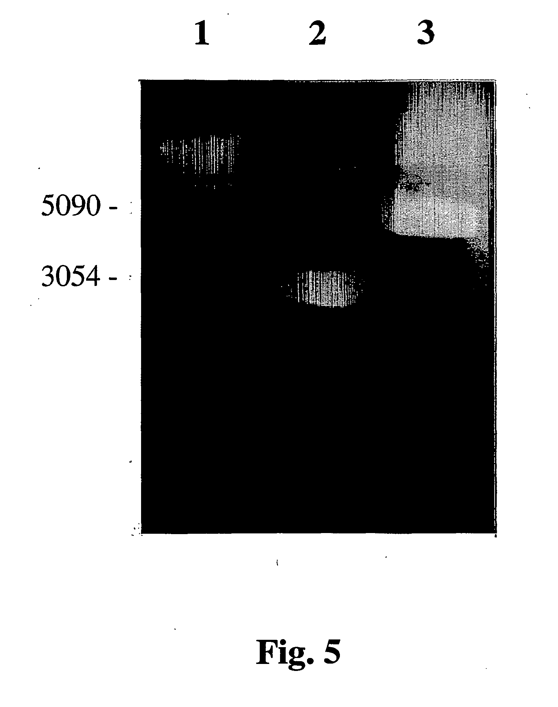

Construction of MpComb3H-K2S. We generated a stop codon between K2S and gp3 in pComb3H-K2S with the aid of the mutagenic primers (MSTPA and MASTPA) (Fig. 4). In order to enrich the newly synthesized and mutated MpComb3H-K2S, the cycle amplification mixture was thoroughly digested with Dpn I to degrade the old dam methylated pComb3H-K2S template (Dpn I prefers dam methylated DNA). After transforming of E. coli XL-1 Blue with MpComb3H-K2S, a transformant XM[K2S] was selected for further study. As a consequence of bp substitution, one Sfi I cleavage site close to the 3' end of K2S gene was lost after site-directed mutagenesis. A linear version of Sfi I cleaved MpComb3H-K2S was observed at 4319 bp without the appearance of inserted K2S gene fragment (Fig. 5, lane 3). Thus, the K2S gene encoding by MpComb3H-K2S was expressed in non-gp3 fusion form in XM[K2S].

Expression and purification of K2S. K2S expression in XM[K2S] was induced by IPTG. r-K2S was detectable by using ELISA both in the periplasmic space and in the culture supernatant The amount of the heterologous protein in each preparation was determined by sandwich ELISA and related to the standard tPA. From 100 ml of the bacterial culture in shaker flask with the O.D. [600 nm] of 50, the periplasmic fraction yielded 1.38 µg of r-K2S (approximately 32%) whereas 2.96 µg of r-K2S (approximately 68%) was obtained in the ammonium precipitated culture supernatant. Sandwich ELISA was used to verify the PEG precipitated phage from VCSM13 infected XM[K2S]. No r-K2S captured by monoclonal anti-kringle 2 antibody was detected by anti-M13 conjugated HRP, indicating that K2S is not presented on the phage particles if gp3 is missing.

Amidolytic activity measurement. If serine protease domain is present in the sample, plasminogen will be converted to plasmin. The produced plasmin will further digest the S-2251 substrate to a colour product, p-nitroaniline, which has a maximum absorbance at 405 nm. The specific activity of the recombinant product is in accordance with the absorbance. The fibrinogen-dependent enzymatic activity of each sample i.e. K2S-ϕ periplasmic r-K2S or culture supernatant r-K2S, was evaluated and compared. Both K2S-ϕ and periplasmic r-K2S illustrated notably low enzymatic activity, which was below the sensitivity of the test (0.25 IU/ml). The culture supernatant r-K2S gave the fibrinogen-dependent enzymatic activity of 7 IU/ml. Thus, from 100 ml culture we obtained a total of 700 IU enzymatic activity. Without fibrinogen no enzymatic activity of the r-K2S purified from culture supernatant was observed - whereas standard melanoma tPA showed some activity.

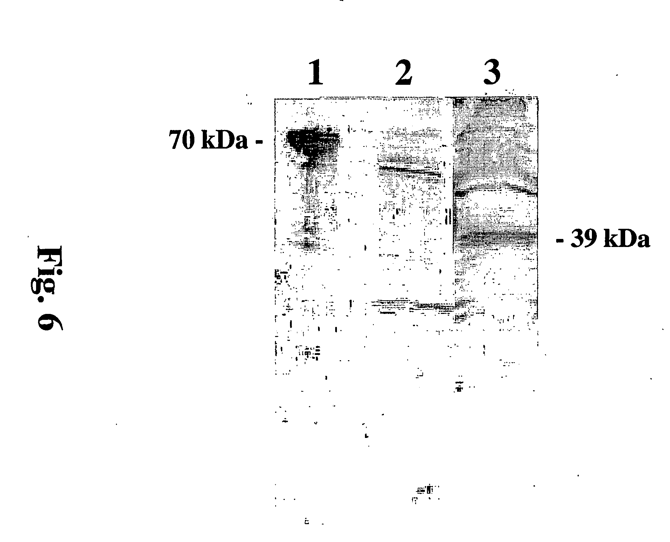

Demonstration of recombinant protein by immunoblotting. Partially purified K2S from culture supernatant of XM[K2S] revealed a molecular mass of 39 kDa by using sheep anti-tPA antibodies (Fig. 6). The negative control, partially purified culture supernatant of non-transformed XL1-Blue, contained no reactive band with a similar size.

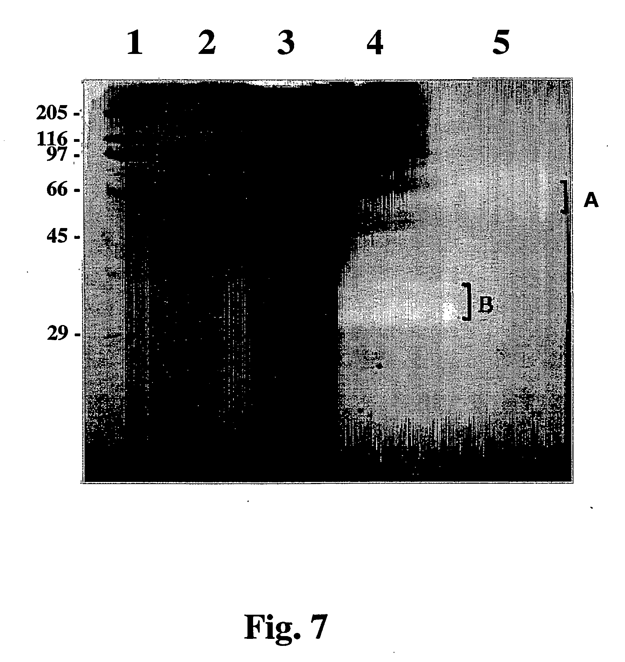

Localization of active enzyme by PAGE. The plasminogen has been copolymerized and immobilized with gelatin in the polyacrylamide gel prior to electrophoresis. The ammonium sulfate precipitated culture supernatants of E. coli XL-1 Blue, E. coli XL-1 Blue transformed with pComb3HSS and XM[K2S] were analyzed (Fig. 7). All samples were processed in nonreducing condition to preserve the correct conformation and activity of the serine protease domain. Transparent areas of serine protease digested plasminogen were observed only in the ammonium sulfate precipitated culture supernatants of XM[K2S] at 34 and 37 kDa postions. The other samples gave no clearing zones. The positive control lane of standard melanoma tPA also demonstrated enzymatic activity at 66 and 72 kDa positions.

### REFERENCES

1. Allen, S., H. Y. Naim, and N. J. Bulleid. 1995. Intracellular folding of tissue-type plasminogen activator. Effects of disulfide bond formation on N-linked glycosylation and secretion. J. Biol. Chem. 270:4797-4804.
2. Ames, G. F., C. Prody, and S. Kustu. 1984. Simple, rapid, and quantitative release of periplasmic proteins by chloroform. J. Bacteriol. 160:1181-1183.
3. Barbas, C. F. III, A. S. Kang, R. A. Lerner, and S. J. Benkovic. 1991. Assembly of combinatorial antibody libraries on phage surfaces: the gene III site. Proc. Natl. Acad. Sci. U. S. A. 88:7978-7982.
4. Barbas, C. F. III, and J. Wagner. 1995. Synthetic human antibodies: selecting and evolving functional proteins. A Companion to Methods in Enzymology 8: 94-103.
5. Bennett, W. F., N. F. Paoni, B. A. Keyt, D. Botstein, A. J. Jones, L. Presta, F. M. Wurm, and M. J. Zoller. 1991. High resolution analysis of functional determinants on human tissue-type plasminogen activator. J Biol Chem. 266:5191-5201.
6. Betton, J. M., N. Sassoon, M. Hofnung, and M. Laurent. 1998. Degradation versus aggregation of misfolded maltose-binding protein in the periplasm of Escherichia coli. J. Biol. Chem. 273:8897-8902.
7. Camiolo, S. M., S. Thorsen, and T. Astrup. 1971. Fibrinogenolysis and fibrinolysis with tissue plasminogen activator, urokinase, streptokinase-activated human globulin and plasmin. Proc. Soc. Exp. Biol. Med. 38:277-280.
8. Cartwright, T. 1992. Production of t-PA from animal cell culture, p. 217-245. In R. E. Spier, and J. B. Griffiths (ed.), Animal Cell Biotechnology, Vol 5. Academic Press, N.Y.
9. Curry, K. A., A. W. Yem, M. R. Deibel, Jr., N. T. Hatzenbuhler, J. G. Hoogerheide, and C. S. Tomich. 1990. Escherichia coli expression and processing of human interleukin-1 beta fused to signal peptides. DNA Cell Biol. 9:167-175.
10. Datar, R. V., T. Cartwright, an C.-G. Rosen. 1993. Process economics of animal cell and bacterial fermentations: a case study analysis of tissue plasminogen activator. Biotechnology 11:349-357.
11. Denefle, P., S. Kovarik, T. Ciora, N. Gosselet, J. C. Benichou, M. Latta, F. Guinet, A. Ryter, and J. F. Mayaux. 1989. Heterologous protein export in Escherichia coli: influence of bacterial signal peptides on the export of human interleukin 1 beta. Gene 85:499-510.
12. Griffiths, J. B., A. Electricwala. 1987. Production of tissue plasminogen activators from animal cells. Adv. Biochem. Eng. Biotechnol. 34:147-166.
13. Harris, T. J., T. Patel, F. A. Marston, S. Little, J. S. Emtage, G. Opdenakker, G. Volckaert , W. Rombauts , A. Billiau, and P. De Somer. 1986. Cloning of cDNA coding for human tissue-type plasminogen activator and its expression in Escherichia coli. Mol. Biol. Med. 3:279-292.
14. Heussen, C., and E. B. Dowdle. 1980. Electrophoretic analysis of plasminogen activators in polyacrylamide gels containing sodium dodecyl sulfate and copolymerized substrates. Anal. Biochem. 102:196-202.
15. Heussen, C., F. Joubert, and E. B. Dowdle. 1984. Purification of human tissue plasminogen activator with Erythrina trypsin inhibitor. J. Biol. Chem. 259:11635-11638.
16. Hu, C. K., U. Kohnert, O. Wilhelm, S. Fischer, and M. Llinas. 1994. Tissue-type plasminogen activator domain-deletion mutant BM 06.022: modular stability, inhibitor binding, and activation cleavage. Biochemistry 33:11760-11766.
17. Kipriyanov, S. M., G. Moldenhauer, and M. Little. 1997. High level production of soluble single chain antibodies in small-scale Escherichia coli cultures. J. Immunol. Methods 200:69-77.
18. Ko, J. H., D. K. Park, I. C. Kim, S. H. Lee, and S. M. Byun. 1995. High-level expression and secretion of streptokinase in Excherichia coli. Biotechnol. Lett. 17:1019-1024.
19. Kouzuma, Y., N. Yamasaki, and M. Kimura. 1997. The tissue-type plasminogen activator inhibitor ETIa from Erythrina variegata: structural basis for the inhibitory activity by cloning, expression, and mutagenesis of the cDNA encoding ETIa. J. Biochem. (Tokyo) 121:456-463.
20. Lasters, I., N . Van Herzeele, H. R. Lijnen, D. Collen, and L. Jespers. 1997. Enzymatic properties of phage-displayed fragments of human plasminogen. Eur. J. Biochem. 244:946-952.
21. Lobel, L. I., P. Rausch, I. Trakht, S. Pollak, and J. W. Lustbader. 1997. Filamentous phage displaying the extracellular domain of the hLH/CG receptor bind hCG specifically. Endocrinology. 138:1232-1239.
22. Lubiniecki, A., R. Arathoon, G. Polastri, J. Thomas, M. Wiebe, R. Gamick, A. Jones, R. van Reis, and S. Builder. Selected strategies for manufacture and control of recombinant tissue plasminogen activator prepared from cell culture, p. 442-451. In R. E. Spier, J. B. Griffiths, J. Stephenne, and P. J. Crooy (ed.), Advances in animal cell biology and technology for bioprocesses. Butterworths, London.
23. Lucic, M. R., B. E. Forbes, S. E. Grosvenor, J. M. Carr, J. C. Wallace, and G. Forsberg. 1998. Secretion in Escherichia coli and phage-display of recombinant insulin-like growth factor binding protein-2. J. Biotechnol. 61:95-108.
24. Martin, U., S. Fischer, U. Kohnert, H. Lill, R. Rudolph, G. Sponer, A. Stem, and K. Strein. 1990. Properties of a novel plasminogen activator (BM 06.022) produced in Escherichia coli. Z. Kardiol. 79:167-170.
25. Obukowicz, M. G., M. E. Gustafson, K. D. Junger, R. M. Leimgruber, A. J. Wittwer, T. C. Wun, T. G. Warren, B. F. Bishop, K. J. Mathis, D. T. McPherson, N. R. Siegel, M. G. Jenning, B. B. Brightwell, J. A. Diaz-Cllier, L. D. Bell, C. S. Craik, and W. C. Tacon. 1990. Secretion of active kringle-2-serine protease in Escherichia coli. Biochemistry 29:9737-9745.
26. Parmley, S. F., and G. P. Smith. 1988. Antibody-selectable filamentous fd phage vectors: affinity purification of target genes. Gene 73:305-318.
27. Pennica, D., W. E. Holmes, W. J. Kohr, R. N. Harkins, G. A. Vehar, C. A. Ward, W. F. Bennett, E. Yelverton, P. H. Seeburg, H. I. Heyneker, D. V. Goeddel, and D. Collen. 1983. Cloning and expression of human tissue-type plasminogen activator cDNA in E. coli. Nature 301:214-221.
28. Rippmann, J. F., M. Klein, C. Hoischen, B. Brocks, W. J. Rettig, J. Gumpert, K. Pfizenmaier, R. Mattes, and D. Moosmayer. 1998. Procaryotic expression of single-chain variable-fragment (scFv) antibodies: secretion in L-form cells of Proteus mirabilis leads to active product and overcomes the limitations of periplasmic expression in Escherichia coli: Appl. Environ. Microbiol. 64:4862-4869.
29. Saito, Y., Y. Ishii, H. Sasaki, M. Hayashi, T. Fujimura, Y. Imai, S. Nakamura, S. Suzuki, J. Notani, T. Asada, H. Horiai, K. Masakazu, and N. Mineo. 1994. Production and characterization of a novel tissue-type plasminogen activator derivative in Escherichia coli. Biotechnol. Prog. 10:472-479.
30. Sarmientos, P., M. Duchesne, P. Denèfle, J. Boiziau, N. Fromage, N. Delporte, F. Parker, Y. Lelièvre, J.-F. Mayaux, and T. Cartwright. 1989. Synthesis and purification of active human tissue plasminogen activator from Escherichia coli. Biotechnology 7:495-501.
31. Scherrer, S., N. Robas, H. Zouheiry, G. Branlant, and C. Branlant. 1994. Periplasmic aggregation limits the proteolytic maturation of the Escherichia coli penicillin G amidase precursor polypeptide. Appl. Microbiol. Biotechnol. 42:85-89.
32. Soeda, S., M. Kakiki, H. Shimeno, and A. Nagamatsu. 1986. Rapid and high-yield purification of porcine heart tissue-type plasminogen activator by heparin-sepharose choromatography. Life Sci. 39:1317-1324.
33. Szarka, S. J., E. G. Sihota, H. R. Habibi., and S.-L. Wong. 1999. Staphylokinase as a plasminogen activator component in recombinant fusion proteins. Appl. Environ. Microbiol. 65:506-513.
34. Waldenström, M., E. Holmgren, A. Attersand, C. Kalderen, B. Lowenadler, B. Raden, L. Hansson, and G. Pohl. 1991. Synthesis and secretion of a fibrinolytically active tissue-type plasminogen activator variant in Escherichia coli. Gene 99:243-248.
35. Wan, E. W.-M., and F. Baneyx. 1998. TolAIII Co-overexpression Facilitates the Recovery of Periplasmic Recombinant Proteins into the Growth Medium of Escherichia coli. Protein Expr. Purif. 14:13-22.
36. Zacharias, U., B. Fischer, F. Noll, and H. Will. 1992. Characterization of human tissue-type plasminogen activator with monoclonal antibodies: mapping of epitopes and binding sites for fibrin and lysine. Thromb. Haemost. 67:88-94.

### FIGURE LEGENDS

FIG. 1. Validation of PCR amplification product of the K2S gene from the p51-3 vector by using SK2/174 and ASSP primers. Lane 1 shows 1 kb marker (Roche Molecular Biochemicals, Indianapolis, IN). Lane 2 was loaded with 1 µl of amplified product. A single band at 1110 bp is depicted. The electrophoresis was performed on a 1% agarose gel.
FIG. 2. Identification of inserted K2S gene at 1110 bp (*) after Sfi I digested pComb3H-K2S was demonstrated in lane 3. Lane 1 shows 1 kb marker. Lane 2 was loaded with uncut pComb3H-K2S. The electrophoresis was performed on a 1% agarose gel.
FIG. 3. Scheme of pComb3H-K2S showing two Sfi I cloning sites into which the K2S gene was inserted. Signal sequence (OmpA), ribosome binding site (RIBS), lac promoter, and gpIII gene are also depicted.
FIG. 4. Schematic diagram of the mutation site at the junction between the K2S and gpIII genes on pComb3H-K2S. The annealing site of pComb3H-K2S is bound with a set of mutation primers (MSTPA and MASTPA) containing modified oligonucleosides (underlined). After performing the cycle amplification, the Sfi I site 1 (in bold) is modified and lost in the newly synthesized strand.
FIG. 5. Characterization of newly synthesized MpComb3H-K2S by the Sfi I restriction enzyme.
A single band at 4319 bp that refers to a single cleavage site of MpComb3H-K2S is observed in lane 3. No inserted K2S band at 1110 bp can be visualized. Lane 1 shows 1 kb marker. Lane 2 was loaded with uncut MpComb3H-K2S. The electrophoresis was performed on a 1% agarose gel.
FIG. 6. Identification of immunological reactive band with of recombinant DNA-derived protein purified from XM[K2S] culture supernatant with sheep anti-tPA conjugated HRP. Lane 1 was loaded with 40 ng of standard melanoma tPA (86/670), which showed the reactive band at 70 kDa. The partially purified and concentrated culture supernatants from non-transformed E. coli XL1- Blue and XM[K2S] were applied to lane 2 and 3 respectively. The distinct reactive band was particularly demonstrated in lane 3 at 39 kDa.
FIG. 7. Molecular weight determination of extracellular r-K2S harboring active serine protease domain by copolymerized plasminogen polyacrylamide gel electrophoresis. Lane 1 contained the indicated molecular weight standards (×10⁻³), SDS-6H (Sigma, Saint Louis, MO). Fifty µg of the 55% saturated ammonium sulfate precipitated culture supernatant of XL-1 Blue, Xl-1 Blue transformed with pComb3HSS, and XM[K2S] were loaded in lane 2, 3, and 4 respectively. Lane 5 contained 50 mIU of standard melanoma tPA (86/670). Transparent zones of digested plasminogen in polyacrylamide gel are visible only in lane 4 at molecular weight of 34 and 37 kDa (B) and lane 5 at molecular weight of 66 and 72 kDa (A).
FIG. 8. Structure A (SEQ ID NO:11)
Native K2S molecule from amino acids 174-527 without modification.

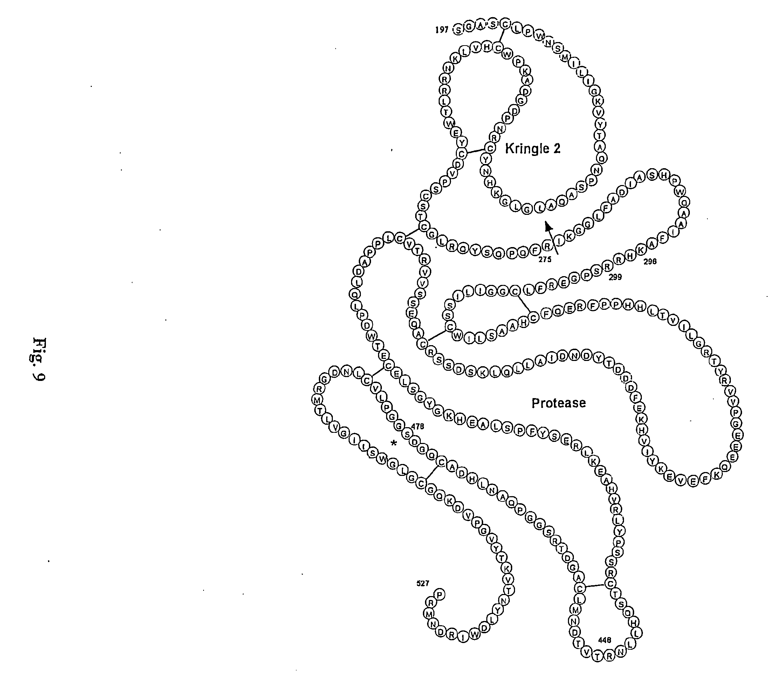

FIG. 9. Structure B-0 (SEQ ID NO:12)
Native K2S molecule from amino acids 197-527 without modification.

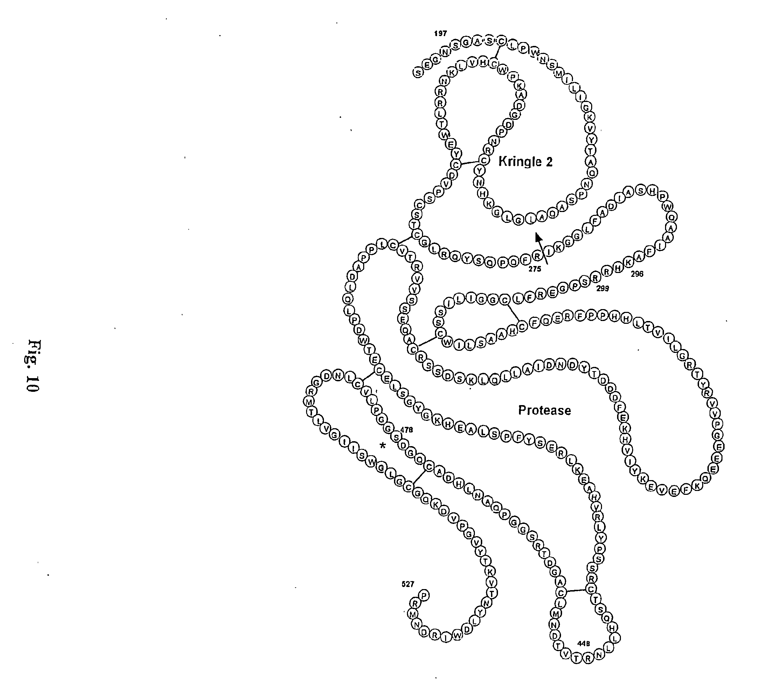

FIG. 10. Structure B-1 (SEQ ID NO:13)
K2S molecule from amino acids 193-527, wherein to Structure B-0 of Fig. 9 the amino acids SEGN were added at the N-terminal portion.

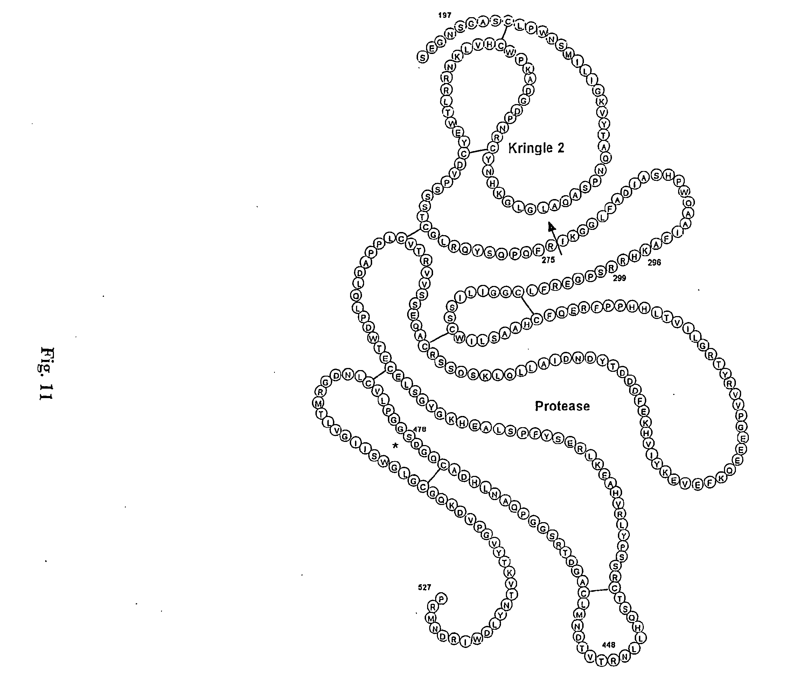

FIG. 11. Structure B-2 (SEQ ID NO:14)
K2S molecule from amino acids 193-527, as in Fig. 10, wherein Cys-261 was exchanged for Ser.
FIG. 12. Structure B-3 (SEQ ID NO:15)
K2S molecule from amino acids 191-527, wherein to Structure B-0 of Fig. 9 the amino acids SEGNSD were added at the N-terminal portion.
FIG. 13. Structure B-4 (SEQ ID NO:16)
K2S molecule from amino acids 191-527, as in Fig. 12, wherein Cys-261 was exchanged for Ser.
FIG. 14. Structure C (SEQ ID NO:17)
Native K2S molecule from amino acids 220-527 without modification. This molecule may be further modified in a similar manner as disclosed for structure B in figures 10-13.
FIG. 15. Structure D (SEQ ID NO:18)
Native K2S molecule from amino acids 260-527 without modification. This molecule may be further modified in a similar manner as disclosed for structure B in figures 10-13.
FIG. 16. tPA molecule (SEQ ID NO:19)

**TABLE 1. Detection of r-K2S molecule in phage preparation by sandwich ELISA**

| Capture antibody | Tracer antibody (conjugated HRP) | | | |
|---|---|---|---|---|
| | Anti-tPA | | Anti-M13 | |
| | K2S-φ | VCSM13^{a} | K2S-φ | VCSM13 |
| Anti-kringle 2^{b} | 1.12 ± 0.04^{c} | 0.12 ± 0.03 | 1.89 ± 0.02 | 0.16 ± 0.02 |
| Anti-M13 | 0.17 ± 0.01 | 0.14 ± 0.05 | 1.91 ± 0.02 | 1.88 ± 0.03 |

| | | | | |
|---|---|---|---|---|
| ^{a} VCSM13 was harvested from XL-1 Blue transformed with pComb3HSS. ^{b} Mouse monoclonal anti-kringle 2 (16/B) was used. The other antibodies were prepared from sheep immunoglobulin. ^{c} Value is mean of absorbance of each sample which was assayed in triplicate. | | | | |

## Claims

1. Method for the production of recombinant DNA-derived tissue plasminogen activator (tPA), a tPA variant, a Kringle 2 Serine protease molecule (K2S) or a K2S variant in prokaryotic cells, wherein said tPA, tPA variant, K2S molecule or K2S variant is secreted extracellularly as an active and correctly folded protein, **characterised in that** the prokaryotic cell contains and expresses a vector comprising the DNA coding for said tPA, tPA variant, K2S molecule or K2S variant operably linked to the DNA coding for the signal peptide OmpA.

2. Method according to claim 1, **characterised in that** said prokaryotic cell contains and expresses a vector comprising the DNA coding for said tPA, tPA variant, K2S molecule or K2S variant operably linked to the DNA coding for the signal peptide OmpA which is operably linked to the nucleic acid molecule defined by the sequence TCTGAGGGAAACAGTGAC (SEQ ID NO:1) or a functional derivative thereof.

3. Method according to claim 1 or 2, **characterised in that** the prokaryotic cell is E. coli.

4. Method according to one of claims 1 to 3, **characterised in that** the the following steps are carried out:
a) the DNA encoding the tPA, tPA variant, K2S molecule or K2S variant is amplified by PCR;
b) the PCR product is purified;
c) said PCR product is inserted into a vector comprising the DNA coding for OmpA signal peptide and the DNA coding for gpIII in such a way that said PCR product is operably linked upstream to the DNA coding for the OmpA signal sequence and linked downstream to the DNA coding for gpIII of said vector;
d) that a stop codon is inserted between said tPA, tPA variant, K2S molecule or K2S variant and gpIII;
e) said vector is expressed by the prokaryotic cell;
f) the tPA, tPA variant, K2S molecule or K2S variant is purified.

5. Method according to one of claims 1 to 4, **characterised in that** the vector is a phagemid vector comprising the DNA coding for OmpA signal peptide and the DNA coding for gpIII.

6. Method according to one of claims 1 to 5, **characterised in that** the vector is the pComb3HSS phagemid.

7. Method according to one of claims 1 to 6, **characterised in that** the DNA Sequence of OmpA linked upstream to K2S comprises the following sequence or a variant due to the degenerate nucleotide code:

8. Method according to one of claims 1 to 7, **characterised in that** the DNA Sequence of OmpA comprises the following sequence:

9. Method according to one of claims 1 to 8, **characterised in that** the DNA Sequence of OmpA consists of the following sequence:

10. Method according to one of claims 1 to 9, **characterised in that** the DNA of the tPA, tPA variant, K2S molecule or K2S variant is preceeded by a lac promotor and/or a ribosomal binding site.

11. Method according to one of claims 1 to 10, **characterised in that** the DNA coding for the tPA, tPA variant, K2S molecule or K2S variant is selected from the group of DNA molecules coding for at least 90% of the amino acids 87 - 527, 174 - 527, 180 - 527 or 220 - 527 of the human tissue plasminogen activator protein as shown in Nature 301 (5897), 214-221 (1983).

12. Method according to one of claims 5 to 11, **characterised in that** the DNA Sequence of K2S comprises the following sequence or a functional variant thereof or a variant due to the degenerate nucleotide code:

13. Method according to one of claims 5 to 12, **characterised in that** the DNA Sequence of K2S consists of the following sequence:

14. DNA molecule **characterised in that** it is coding for:
a) a DNA coding for the OmpA signal peptide
b) a DNA sequence coding for a polypeptide containing the amino acids SEGN or SEGNSD and
c) a DNA sequence coding for a polypeptide containing the kringle 2 domain and the serine protease domain of tissue plasminogen activator protein,
wherein said DNA coding the OmpA signal peptide of a) is fused to the DNA sequence coding for the short peptide **characterised by** the amino acid sequences SEGN or SEGNSD of b) and located at the N-terminal portion of the kringle 2 domain and the serine protease domain of tissue plasminogen activator protein of c).

15. DNA molecule according to claim 14, **characterised in that** said DNA sequence comprises the following sequence or a variant due to the degenerate nucleotide code:

16. DNA molecule according to claim 14 or 15, **characterised in that** said DNA sequence consists of the following sequence:

17. DNA molecule according to one of claims 14 to 16, **characterised in that** said DNA sequence c) is coding for at least 90% of the amino acids 87 - 527 of the human tissue plasminogen activator protein as shown in Nature 301 (5897), 214-221 (1983).

18. DNA molecule according to one of claims 14 to 17, **characterised in that** said DNA sequence c) is coding for at least 90% of the amino acids 174 - 527 of the human tissue plasminogen activator protein as shown in Nature 301 (5897), 214-221 (1983).

19. DNA molecule according to any one of claims 14 to 18, **characterised in that** said DNA sequence c) is coding for at least 90% of the amino acids 180 - 527 of the human tissue plasminogen activator protein as shown in Nature 301 (5897), 214-221 (1983).

20. DNA molecule according to any one of claims 14 to 19, **characterised in that** said DNA sequence c) is coding for at least 90% of the amino acids 220 - 527 of the human tissue plasminogen activator protein as shown in Nature 301 (5897), 214-221 (1983).

21. DNA molecule according to any one of claims 14 to 20 **characterised in that** said DNA sequence c) is hybridizing under stringent conditions to the following sequence:

22. DNA molecule according to any one of claims 14 to 21, **characterised in that** said DNA sequence c) consists of the following sequence:

23. Fusion protein of OmpA and K2S, **characterised in that** it comprises a protein **characterised by** the following amino acid sequence:

24. Fusion protein of OmpA and K2S according to claim 23, **characterised in that** it consists of a protein **characterised by** the following amino acid sequence:

25. A vector containing a DNA sequence according to any one of claims 14 to 22.

26. A vector according to claim 25, wherein said DNA sequence is preceded by a lac promoter and a ribosomal binding site.

27. The vector pComb3HSS containing a DNA according to any one of claims 14 to 22, wherein the expression of the gp III protein is suppressed or inhibited by deleting the DNA molecule encoding said gp III protein or by a stop codon between the gene coding for a polypeptide containing the kringle 2 domain and the serine protease domain of tissue plasminogen activator protein and the protein III gene.

28. A prokaryotic host cell comprising a DNA molecule according to any one of claims 14 to 22.

29. A prokaryotic host cell comprising a vector according to any one of claims 25 to 27.

30. An E. coli host cell comprising a DNA molecule according to any one of claims 14 to 22.

31. An E. coli host cell comprising a vector according to any one of claims 25 to 27.

32. Use of a DNA molecule according to any one of claims 14 to 22 or of a vector according to any one of claims 26 to 28 or a host cell according to any one of claims 28 to 31 in a method for the production of a polypeptide having the activity of tissue plasminogen activator.

33. Use according to claim 32, wherein said method is a method according to any one of claims 1 to 13.

## Patentansprüche

1. Verfahren zur Herstellung eines rekombinanten, von DNA abgeleiteten Gewebe-Plasminogen-Aktivators (tPA), einer tPA-Variante, eines Kringle-2-Serin-protease-Moleküls (K2S) oder einer K2S-Variante in prokaryontischen Zellen, wobei der tPA, die tPA-Variante, das K2S-Molekül oder die K2S-Variante extrazellulär als ein aktives und korrekt gefaltetes Protein sezerniert werden, **dadurch gekennzeichnet, dass** die prokaryontische Zelle einen Vektor enthält und exprimiert, der die für den tPA, die tPA-Variante, das K2S-Molekül oder die K2S-Variante kodierende DNA in funktioneller Verknüpfung mit der für das Signalpeptid OmpA kodierenden DNA umfasst.

2. Verfahren nach Anspruch 1, **dadurch gekennzeichnet, dass** die prokaryontische Zelle einen Vektor enthält und exprimiert, der die für den tPA, die tPA-Variante, das K2S-Molekül oder die K2S-Variante kodierende DNA in funktioneller Verknüpfung mit der für das Signalpeptid OmpA kodierenden DNA umfasst, wobei das Signalpeptid funktionell mit dem durch die Sequenz TCTGAGGGAAACAGTGAC (SEQ ID NO: 1) definierten Nucleinsäuremolekül oder einem funktionellen Derivat davon verknüpft ist.

3. Verfahren nach Anspruch 1 oder 2, **dadurch gekennzeichnet, dass** es sich bei der prokaryontischen Zelle um E. coli handelt.

4. Verfahren nach einem der Ansprüche 1 bis 3, **dadurch gekennzeichnet, dass** die folgenden Stufen durchgeführt werden:
a) die für den tPA, die tPA-Variante, das K2S-Molekül oder die K2S-Variante kodierende DNA wird durch PCR amplifiziert;
b) das PCR-Produkt wird gereinigt;
c) das PCR-Produkt wird in einen Vektor, der die für das OmpA-Signalpeptid kodierende DNA und die für gplll kodierende DNA umfasst, auf solche Weise inseriert, dass das PCR-Produkt funktionell strangaufwärts mit der für die OmpA-Signalsequenz kodierenden DNA und strangabwärts mit der für gplll kodierenden DNA dieses Vektors verknüpft ist;
d) ein Stoppcodon wird zwischen den tPA, die tPA-Variante, das K2S-Molekül oder die K2S-Variante und gpIII inseriert;
e) der Vektor wird durch die prokaryontische Zelle exprimiert; und
f) der tPA, die tPA-Variante, das K2S-Molekül oder die K25-Variante werden gereinigt.

5. Verfahren nach einem der Ansprüche 1 bis 4, **dadurch gekennzeichnet, dass** es sich bei dem Vektor um einen Phagemid-Vektor handelt, der die für das OmpA-Signalpeptid kodierende DNA und die für gpIII kodierende DNA umfasst.

6. Verfahren nach einem der Ansprüche 1 bis 5, **dadurch gekennzeichnet, dass** es sich bei dem Vektor um das pComb3HSS-Phagemid handelt.

7. Verfahren nach einem der Ansprüche 1 bis 6, **dadurch gekennzeichnet, dass** die DNA-Sequenz von OmpA, die strangaufwärts mit K2S verknüpft ist, die folgende Sequenz oder eine Variante davon aufgrund des degenerierten Nucleotidcodes umfasst:

8. Verfahren nach einem der Ansprüche 1 bis 7, **dadurch gekennzeichnet, dass** die DNA-Sequenz von OmpA die folgende Sequenz umfasst:

9. Verfahren nach einem der Ansprüche 1 bis 8, **dadurch gekennzeichnet, dass** die DNA-Sequenz von OmpA aus der folgenden Sequenz besteht:

10. Verfahren nach einem der Ansprüche 1 bis 9, **dadurch gekennzeichnet, dass** der DNA des tPA, der tPA-Variante, des K2S-Moleküls oder der K2S-Variante ein lac-Promotor und/oder eine ribosomale Bindungsstelle vorausgehen.

11. Verfahren nach einem der Ansprüche 1 bis 10, **dadurch gekennzeichnet, dass** die für den tPA, die tPA-Variante, das K2S-Molekül oder die K2S-Variante kodierende DNA aus der Gruppe von DNA-Molekülen ausgewählt ist, die für mindestens 90 % der Aminosäuren 87-527, 174-527, 180-527 oder 220-527 des humanen Gewebe-Plasminogen-Aktivator-Proteins kodieren, wie in Nature, Bd. 301 (5897) (1983), S. 214-221, gezeigt.

12. Verfahren nach einem der Ansprüche 5 bis 11, **dadurch gekennzeichnet, dass** die DNA-Sequenz von K2S die folgende Sequenz oder eine funktionelle Variante davon oder eine Variante davon aufgrund des degenerierten Nucleotidcodes umfasst:

13. Verfahren nach einem der Ansprüche 5 bis 12, **dadurch gekennzeichnet, dass** die DNA-Sequenz von K2S aus der folgenden Sequenz besteht:

14. DNA-Molekül, **dadurch gekennzeichnet, dass** es kodiert für:
a) eine für das OmpA-Signalpeptid kodierende DNA,
b) eine DNA-Sequenz, die für ein Polypeptid kodiert, das die Aminosäuren SEGN oder SEGNSD enthält, und
c) eine DNA-Sequenz, die für ein Polypeptid kodiert, das die Kringle-2-Domäne und die Serin-protease-Domäne von Gewebe-Plasminogen-Aktivator-Protein enthält,
wobei die für das OmpA-Signalpeptid kodierende DNA von a) mit der für das kurze Peptid kodierenden DNA-Sequenz fusioniert ist, die durch die Aminosäuresequenzen SEGN oder SEGNSD von b) charakterisiert ist und sich am N-terminalen Bereich der Kringle-2-Domäne und der Serin-Protease-Domäne von Gewebe-Plasminogen-Aktivator-Protein von c) befindet.

15. DNA-Molekül nach Anspruch 14, **dadurch gekennzeichnet, dass** die DNA-Sequenz die folgende Sequenz oder eine Variante davon aufgrund des degenerierten Nucleotidcodes umfasst:

16. DNA-Molekül nach Anspruch 14 oder 15, **dadurch gekennzeichnet, dass** die DNA-Sequenz aus der folgenden Sequenz besteht:

17. DNA-Molekül nach einem der Ansprüche 14 bis 16, **dadurch gekennzeichnet, dass** die DNA-Sequenz c) für mindestens 90 % der Aminosäuren 87-527 des humanen Gewebe-Plasminogen-Aktivator-Proteins kodiert, wie in Nature, Bd. 301 (5897) (1983), S. 214-221, gezeigt.

18. DNA-Molekül nach einem der Ansprüche 14 bis 17, **dadurch gekennzeichnet, dass** die DNA-Sequenz c) für mindestens 90 % der Aminosäuren 174-527 des humanen Gewebe-Plasminogen-Aktivator-Proteins kodiert, wie in Nature, Bd. 301 (5897) (1983), S. 214-221, gezeigt.

19. DNA-Molekül nach einem der Ansprüche 14 bis 18, **dadurch gekennzeichnet, dass** die DNA-Sequenz c) für mindestens 90 % der Aminosäuren 180-527 des humanen Gewebe-Plasminogen-Aktivator-Proteins kodiert, wie in Nature, Bd. 301 (5897) (1983), S. 214-221, gezeigt.

20. DNA-Molekül nach einem der Ansprüche 14 bis 19, **dadurch gekennzeichnet, dass** die DNA-Sequenz c) für mindestens 90 % der Aminosäuren 220-527 des humanen Gewebe-Plasminogen-Aktivator-Proteins kodiert, wie in Nature, Bd. 301 (5897) (1983), S. 214-221, gezeigt.

21. DNA-Molekül nach einem der Ansprüche 14 bis 20, **dadurch gekennzeichnet, dass** die DNA-Sequenz c) unter stringenten Bedingungen mit der folgenden Sequenz hybridisiert:

22. DNA-Molekül nach einem der Ansprüche 14 bis 21, **dadurch gekennzeichnet, dass** die DNA-Sequenz c) aus der folgenden Sequenz besteht:

23. Fusionsprotein von OmpA und K2S, **dadurch gekennzeichnet, dass** es ein Protein umfasst, das durch die folgende Aminosäuresequenz charakterisiert ist:

24. Fusionsprotein von OmpA und K2S nach Anspruch 23, **dadurch gekennzeichnet, dass** es aus einem Protein besteht, das durch die folgende Aminosäuresequenz charakterisiert ist:

25. Vektor, enthaltend eine DNA-Sequenz nach einem der Ansprüche 14 bis 22.

26. Vektor nach Anspruch 25, wobei der DNA-Sequenz ein lac-Promotor und eine ribosomale Bindungsstelle vorausgehen.

27. Vektor pComb3HSS, enthaltend eine DNA nach einem der Ansprüche 14 bis 22, wobei die Expression des gpIII-Proteins durch Deletion des für das gpIII-Protein kodierenden DNA-Moleküls oder durch ein Stoppcodon zwischen dem Gen, das für ein Polypeptid mit einem Gehalt an der Kringle-2-Domäne und der Serin-Protease-Domäne von Gewebe-Plasminogen-Aktivator-Protein kodiert, und dem Protein III-Gen unterdrückt oder gehemmt wird.

28. Prokaryontische Wirtszelle, umfassend ein DNA-Molekül nach einem der Ansprüche 14 bis 22.

29. Prokaryontische Wirtszelle, umfassend einen Vektor nach einem der Ansprüche 25 bis 27.

30. E. coli-Wirtszelle, umfassend ein DNA-Molekül nach einem der Ansprüche 14 bis 22.

31. E. coli-Wirtszelle, umfassend einen Vektor nach einem der Ansprüche 25 bis 27.

32. Verwendung eines DNA-Moleküls nach einem der Ansprüche 14 bis 22 oder eines Vektors nach einem der Ansprüche 26 bis 28 oder einer Wirtszelle nach einem der Ansprüche 28 bis 31 in einem Verfahren zur Herstellung eines Polypeptids mit der Aktivität von Gewebe-Plasminogen-Aktivator.

33. Verwendung nach Anspruch 32, wobei es sich bei dem Verfahren um ein Verfahren nach einem der Ansprüche 1 bis 13 handelt.

## Revendications

1. Méthode de production de l'activateur tissulaire du plasminogène (tPA) dérivé d'un ADN recombinant, d'un variant de tPA, d'une molécule Kringle 2 sérine protéase (K2S) ou d'un variant de K2S dans des cellules procaryotes, ledit tPA, ledit variant de tPA, ladite molécule K2S ou ledit variant de K2S étant sécrété hors de la cellule sous la forme d'une protéine active et correctement repliée, **caractérisée en ce que** la cellule procaryote contient et exprime un vecteur comprenant l'ADN codant ledit tPA, ledit variant de tPA, ladite molécule K2S ou ledit variant de K2S lié de manière fonctionnelles l'ADN codant le peptide signal OmpA.

2. Méthode selon la revendication 1, **caractérisée en ce que** ladite cellule procaryote contient et exprime un vecteur comprenant l'ADN codant ledit tPA, ledit variant de tPA, ladite molécule K2S ou ledit variant de K2S lié de manière fonctionnelle à l'ADN codant le peptide signal OmpA qui est lié de manière fonctionnelle à la molécule d'acide nucléique définie par la séquence TCTGAGGGAAACAGTGAC (SEQ ID NO : 1) ou à l'un de ses dérivés fonctionnels.

3. Méthode selon la revendication 1 ou 2, **caractérisée en ce que** la cellule procaryote est *E. coli*.

4. Méthode selon l'une quelconque des revendications 1 à 3, **caractérisée en ce que** les étapes suivantes sont mises en oeuvre :
a) l'ADN codant le tPA, le variant de tPA, la molécule K2S ou le variant de K2S est amplifié par PCR ;
b) le produit PCR; est purifié ;
c) ledit produit PCR est inséré dans un vecteur comprenant l'ADN codant le peptide signal OmpA et l'ADN codant gpIII de manière à ce que ledit produit PCR soit lié de manière fonctionnelle en amont de l'ADN codant la séquence signal de l'OmpA et lié en aval à l'ADN codant gpIII dudit vecteur ;
d) un codon d'arrêt est inséré entre ledit tPA, ledit variant de tPA, ladite molécule K2S ou ledit variant de K2S et gpIII ;
e) ledit vecteur est exprimé par la cellule procaryote ;
f) le tPA, le variant de tPA, la molécule K2S ou le variant de K2S est purifié.

5. Méthode selon l'une quelconque des revendications 1 à 4, **caractérisée en ce que** le vecteur est un vecteur phagemide comprenant l'ADN codant le peptide signal OmpA et l'ADN codant gpIII.

6. Méthode selon l'une quelconque des revendications 1 à 5, **caractérisée en ce que** le vecteur est le phagemide pComb3HSS.

7. Méthode selon l'une quelconque des revendications 1 à 6, **caractérisée en ce que** la séquence d'ADN de l'OmpA liée en amont à K2S comprend la séquence suivante ou un variant attribuable au code dégénéré des nucléotides :

8. Méthode selon l'une quelconque des revendications 1 à 7, **caractérisée en ce que** la séquence d'ADN de l'OmpA comprend la séquence suivante :

9. Méthode selon l'une quelconque des revendications 1 à 8, **caractérisée en ce que** la séquence d'ADN de l'OmpA consiste en la séquence suivante :

10. Méthode selon l'une quelconque des revendications 1 à 9, **caractérisée en ce que** l'ADN du tPA, du variant de tPA, de la molécule K2S ou du variant de K2S est précédé par un promoteur lac et/ou un site de liaison ribosomique.

11. Méthode selon l'une quelconque des revendications 1 à in, **caractérisée en ce que** l'ADN codant le tPA, le variant de tPA, la molécule K2S ou le variant de K2S est choisi dans le groupe des molécules d'ADN codant au moins 90 % des acides aminés 87 à 527, 174 à 527, 180 à 527 ou 220 à 527 de la protéine humaine d'activateur tissulaire du plasminogène, comme décrit dans Nature 301 (5897), 214-221 (1983).

12. Méthode selon l'une quelconque des revendications 5 à 11, **caractérisée en ce que** la séquence d'ADN de K2S comprend la séquence suivante ou l'un de ses variants fonctionnels ou un variant attribuable au code dégénéré des nucléotides :

13. Méthode selon l'une quelconque des revendications 5 à 12, **caractérisée en ce que** la séquence d'ADN de K2S consiste en la séquence suivante :

14. Molécule d'ADN **caractérisée en ce qu'**elle code :
a) un ADN codant le peptide signal OmpA
b) une séquence d'ADN codant un polypeptide contenant les acides aminés SEGN ou SEGNSD et
c) une séquence d'ADN codant un polypeptide contenant le domaine kringle 2 et le domaine sérine protéase de la protéine d'activateur tissulaire du plasminogène,
dans laquelle ledit ADN codant le peptide signal OmpA de a) est fusionné à la séquence d'ADN codant le peptide court **caractérisé par** les séquences d'acides aminés SEGN ou SEGNSD de b) et situé au niveau de la partie N-terminale du domaine kringle 2 et du domaine sérine protéase de la protéine d'activateur tissulaire du plasminogène de c).

15. Molécule d'ADN selon la revendication 14, **caractérisée en ce que** ladite séquence d'ADN comprend la séquence suivante ou un variant attribuable au code dégénéré des nucléotides :

16. Molécule d'ADN selon la revendication 14 ou 15, **caractérisée en ce que** ladite séquence d'ADN consiste en la séquence suivante :

17. Molécule d'ADN selon l'une quelconque des revendications 14 à 16, **caractérisée en ce que** ladite séquence d'ADN c) code au moins 90 % des acides aminés 87 à 527 de la protéine humaine d'activateur tissulaire du plasminogène, comme décrit dans Nature 301 (5897), 214-221 (1983).

18. Molécule d'ADN selon l'une quelconque des revendications 14 à 17, **caractérisée en ce que** ladite séquence d'ADN c) code au moins 90 % des acides aminés 174 à 527 de la protéine humaine d'activateur tissulaire du plasminogène, comme décrit dans Nature 301 (5897), 214-221 (1983).

19. Molécule d'ADN selon l'une quelconque des revendications 14 à 18, **caractérisée en ce que** ladite séquence d'ADN c) code au moins 90 % des acides aminés 180 à 527 de la protéine humaine d'activateur tissulaire du plasminogène, comme décrit dans Nature 301 (5897), 214-221 (1983).

20. Molécule d'ADN selon l'une quelconque des revendications 14 à 19, **caractérisée en ce que** ladite séquence d'ADN c) code au moins 90 % des acides aminés 220 à 527 de la protéine humaine d'activateur tissulaire du plasminogène, comme décrit dans Nature 301 (5897), 214-221 (1983).

21. Molécule d'ADN selon l'une quelconque des revendications 14 à 20, **caractérisée en ce que** ladite séquence d'ADN c) s'hybride dans des conditions stringentes à la séquence suivante :

22. Molécule d'ADN selon l'une quelconque des revendications 14 à 21, **caractérisée en ce que** ladite séquence d'ADN c) consiste en la séquence suivante :

23. Protéine de fusion entre OmpA et K2S, **caractérisée en ce qu'**elle comprend une protéine **caractérisée par** la séquence d'acides aminés suivante :

24. Protéine de fusion entre OmpA et K2S selon la revendication 23, **caractérisée en ce qu'**elle consiste en une protéine **caractérisée par** la séquence d'acides aminés suivante :

25. Vecteur contenant une séquence d'ADN selon l'une quelconque des revendications 14 à 22.

26. Vecteur selon la revendication 25, dans lequel ladite séquence d'ADN est précédée d'un promoteur lac et d'un site de liaison ribosomique.

27. Vecteur pComb3HSS contenant un ADN selon l'une quelconque des revendications 14 à 22, dans lequel l'expression de la protéine gp III est supprimée ou inhibée par la délétion de la molécule d'ADN codant ladite protéine gp III ou par un codon d'arrêt entre le gène codant un polypeptide contenant le domaine kringle 2 et le domaine sérine protéase de la protéine d'activateur tissulaire du plasminogène et le gène de la protéine III.

28. Cellule procaryote hôte comprenant une molécule d'ADN selon l'une quelconque des revendications 14 à 22.

29. Cellule procaryote hôte comprenant un vecteur selon l'une quelconque des revendications 25 à 27.

30. Cellule hôte d'*E. coli* comprenant une molécule d'ADN selon l'une quelconque des revendications 14 à 22.

31. Cellule hôte d'E. coli comprenant un vecteur selon l'une quelconque des revendications 25 à 27.

32. Utilisation d'une molécule d'ADN selon l'une quelconque des revendications 14 à 22 ou d'un vecteur selon l'une quelconque des revendications 26 à 28 ou d'une cellule hôte selon l'une quelconque des revendications 28 à 31 dans une méthode de production d'un polypeptide présentant l'activité de l'activateur tissulaire du plasminogène.

33. Utilisation selon la revendication 32, dans laquelle ladite méthode est une méthode selon l'une quelconque des revendications 1 à 13.
